# EUROPEAN PATENT APPLICATION

(11) **EP 1 840 123 A1**
(43) Date of publication of application: **03.10.2007**
(21) Application number: 07105020.7
(22) Date of filing: 27.03.2007
(51) Int. Cl.: C07D 261/20, C07D 413/12, C07D 498/10, A61K 31/423, A61K 31/424, A61P 11/00, A61P 29/00

(54) **Isoxazolines and their use as inhibitors of phosphodiesterase type-IV**

(30) Priority: 27.03.2006 US 390491
(71) Applicant: Ranbaxy Laboratories Limited, Gurgaon - 122001, Haryana (IN)
(72) Inventor: Balachandran, Sarala D-1302,, 400063, Mumbai (IN); Gupta, Nidhi, 110018, New Delhi (IN); Muthukaman, Nagarajan, 638151, Erode Dt, Tamil Nadu (IN); Khairnar, Vinayak Vasantrao Plot No-31, Dist-Nashik, PIN - 423106 (IN); Ramnani, Sarika House No. 702, Lucknow, U.P., xxxx (IN); Baregama, Lalit K., 312202, Kapasan (IN); Chakladar, Saswati, Dehli, 110095 (IN); Ramaiah, Mandadapu Raghu, 522018, Andra Pradesh (IN); Agarwal, Ritu, PIN - 713211, West Bengal (IN); Dastidar, Sunanda G., 110044, New Dehli (IN); Ray, Abhijit, 110070, New Dehli (IN)
(74) Representative: Cronin, Brian Harold John

(57) **Abstract**

The present invention relates to isoxazoline derivatives of formula I, which can be used as selective inhibitors of phosphodiesterase (PDE) type IV. In particular, compounds disclosed herein can be useful in the treatment of AIDS, asthma, arthritis, bronchitis, chronic obstructive pulmonary disease (COPD), psoriasis, allergic rhinitis, shock, atopic dermatitis, Crohn's disease, adult respiratory distress syndrome (ARDS), eosinophilic granuloma, allergic conjunctivitis, osteoarthritis, ulcerative colitis and other inflammatory diseases in a patient, particularly in humans. The present invention also relates to processes for the preparation of disclosed compounds, as well as pharmaceutical compositions thereof, and their use as phosphodiesterase (PDE) type IV inhibitors.

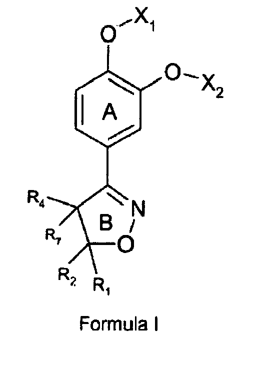

## Description

### Field of Invention

The present invention relates to isoxazoline derivatives, which can be used as selective inhibitors of phosphodiesterase (PDE) type IV. In particular, compounds disclosed herein can be useful in the treatment of AIDS, asthma, arthritis, bronchitis, chronic obstructive pulmonary disease (COPD), psoriasis, allergic rhinitis, shock, atopic dermatitis, Crohn's disease, adult respiratory distress syndrome (ARDS), eosinophilic granuloma, allergic conjunctivitis, osteoarthritis, ulcerative colitis and other inflammatory diseases in a patient, particularly in humans. The present invention also relates to processes for the preparation of disclosed compounds, as well as pharmaceutical compositions thereof, and their use as phosphodiesterase (PDE) type IV inhibitors.

### Background of Invention

It is known that cyclic adenosine-3',5'-monophosphate (cAMP) exhibits an important role of acting as an intracellular secondary messenger. The intracellular hydrolysis of cAMP to adenosine 5'-monophosphate (AMP) causes a number of inflammatory conditions, which include, but are not limited to, psoriasis, allergic rhinitis, shock, atopic dermatitis, Crohn's disease, adult respiratory distress syndrome (ARDS), eosinophilic granuloma, allergic conjunctivitis, osteoarthritis, and ulcerative colitis. Cyclic nucleotide phosphodiesterases (PDE), a biochemically and functionally, highly variable superfamily of the enzyme, is the most important factor in the control of cAMP (as well as of cGMP) levels. Eleven distinct families with more than 25 gene products are currently recognized. Although PDE I, PDE II, PDE III, PDE IV, and PDE VII all use cAMP as a substrate, only the PDE IV and PDE VII types are highly selective for hydrolysis of cAMP. Accordingly, inhibitors of PDE, particularly PDE IV inhibitors, such as rolipram or Ro-1724, are known as cAMP-enhancers. Immune cells contain PDE IV and PDE III, of which PDE IV is prevalent in human mononuclear cells. Thus, the inhibition of phosphodiesterase type IV has been a target for modulation and, accordingly, for therapeutic intervention in a range of disease processes.

The initial observation that xanthine derivatives, theophylline and caffeine inhibit the hydrolysis of cAMP led to the discovery of the required hydrolytic activity in the cyclic nucleotide phosphodiesterase (PDE) enzymes. More recently, distinct classes of PDE have been recognized, and their selective inhibition has led to improved drug therapy. Thus, it was recognized that inhibition of PDE IV could lead to inhibition of inflammatory mediator release and airway smooth muscle relaxation.

3-Aryl-2-isoxazoline derivatives are known as anti-inflammatory agents and isoxazoline compounds are known as inhibitors of TNF release. However, there remains a need for new selective inhibitors of phosphodiesterase (PDE) type IV.

### Summary of Invention

The present invention provides isoxazoline derivatives, which can be used for the treatment of AIDS, asthma, arthritis, bronchitis, chronic obstructive pulmonary disease (COPD), psoriasis, allergic rhinitis, shock, atopic dermatitis, Crohn's disease, adult respiratory distress syndrome (ARDS), eosinophilic granuloma, allergic conjunctivitis, osteoarthritis, ulcerative colitis and other inflammatory diseases, and the processes for the synthesis of these compounds.

Pharmaceutically acceptable salts, pharmaceutically acceptable solvates, enantiomers, diastereomers or N-oxides of these compounds having the same type of activity are also provided.

Pharmaceutical compositions containing compounds described herein, which may also contain pharmaceutically acceptable carriers or diluents, can be used for the treatment of AIDS, asthma, arthritis, bronchitis, chronic obstructive pulmonary disease (COPD), psoriasis, allergic rhinitis, shock, atopic dermatitis, Crohn's disease, adult respiratory distress syndrome, eosinophilic granuloma, allergic conjunctivitis, osteoarthritis, ulcerative colitis and other inflammatory diseases.

In one aspect, provided are compounds having the structure of Formula I, 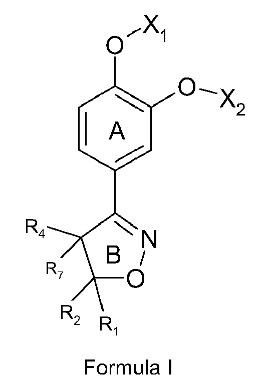 or its pharmaceutically acceptable salts, pharmaceutically acceptable solvates, enantiomers, diastereomers or N-oxides, wherein
- R₁ and R₂: can together form an optionally substituted cycloalkyl or heterocyclyl ring, wherein the optional substituent can be oxo, alkyl, alkenyl, alkynyl, halogen, nitro, -NH₂, -C(=O)NRₓR_{y}, -NHCOOR₆, cyano, hydroxy, alkoxy, or substituted amino;
- R₄: can be hydrogen, alkyl, hydroxy, halogen or carboxy;
- R₇: can be hydrogen or alkyl;
- X₁ and X₂: can be hydrogen, alkyl, cycloalkyl, alkaryl, cycloalkylalkyl, heteroaryl, heterocyclyl, heteroarylalkyl, heterocyclylalkyl, -SO₂R₅, -(CH₂)_{g}NHCOOalkyl, -(CH₂)_{g}C(=O)NRₓR_{y} or -(CH₂)_{g1}C(=O)OR₃ (wherein g can be an integer from 0-3 and g₁ can be an integer from 1-3);
- X₁ and X₂: can together optionally form a cyclic ring fused with ring A of Formula I, wherein ring A contains 3-5 carbon atoms and 2-3 heteroatoms selected from N, O or S;
wherein
R₃ can be alkyl, cycloalkyl or heterocyclyl;
halogen can be F, Cl, Br, or I;
Rₓ and R_{y} each independently can be hydrogen, alkyl, C₃-C₆ alkenyl, C₃-C₆ alkynyl, carboxy, cycloalkyl, -S(O)ₘR₅, aryl, alkaryl, heteroaryl, heterocyclyl, heteroarylalkyl or heterocyclylalkyl;
m can be an integer between 0-2;
R₆ can be alkyl, alkenyl, alkynyl, cycloalkyl, alkaryl, heteroarylalkyl or heterocyclylalkyl; and
R₅ can be hydrogen, alkyl, alkenyl, alkynyl, aryl, cycloalkyl, alkaryl, heteroaryl, heteroarylalkyl, heterocyclyl or heterocyclylalkyl.

In another aspect, provided are compounds selected from:
7-[3-(2,3-dihydro-1H inden-2-yloxy)-4-methoxyphenyl]-5-oxa-6-azaspiro[3.4]oct-6-ene (Compound No. 1);
2-(2,3-dihydro-1H-inden-2-yloxy)-4-(5-oxa-6-azaspiro[3.4]oct-6-en-7-yl)phenol (Compound No. 2);
3-[3-(2,3-dihydro-1*H*-inden-2-yloxy)-4-propoxyphenyl]-1-oxa-2-azaspiro[4.4]non-2-ene (Compound No. 3);
3-[3-(2,3-dihydro-1*H*-inden-2-yloxy)-4-isopropoxyphenyl]-1-oxa-2-azaspiro[4.4]non-2-ene (Compound No. 4);
2-(2,3-dihydro-1*H*-inden-2-yloxy)-4-(1-oxa-2-azaspiro[4.4]non-2-en-3-yl)phenol (Compound No. 5);
3-[3-(2,3-Dihydro-1*H*-inden-2-yloxy)-4-isobutoxyphenyl]-1-oxa-2-azaspiro[4.4]non-2-ene (Compound No. 6);
7-[3-(2,3-Dihydro-1*H*-inden-2-yloxy)-4-ethoxyphenyl]-5-oxa-6-azaspiro[3.4]oct-6-ene (Compound No. 7);
2-(2,3-Dihydro-1*H*-inden-2-yloxy)-4-(1-oxa-2-azaspiro[4.5]dec-2-en-3-yl)phenol (Compound No. 8)
3-[4-Butoxy-3-(2,3-dihydro-1*H*-inden-2-yloxy)phenyl]-1-oxa-2-azaspiro[4.5]dec-2-ene (Compound No. 9);
3-[3-(2,3-Dihydro-1*H*-inden-2-yloxy)-4-propoxyphenyl]-1-oxa-2-azaspiro[4.5]dec-2-ene (Compound No. 10);
3-[4-(Difluoromethoxy)-3-(2,3-dihydro-1*H*-inden-2-yloxy)phenyl]-1-oxa-2-azaspiro[4.5]dec-2-ene (Compound No. 11);
3-[3-(2,3-Dihydro-1*H*-inden-2-yloxy)-4-ethoxyphenyl]-1-oxa-2-azaspiro[4.5]dec-2-ene (Compound No. 12);
3-[4-(Cyclopropylmethoxy)-3-(2,3-dihydro-1*H*-inden-2-yloxy)phenyl]-1-oxa-2-azaspiro[4.5]dec-2-ene (Compound No. 13);
3-[3-(2,3-Dihydro-1*H*-inden-2-yloxy)-4-isopropoxyphenyl]-1-oxa-2-azaspiro[4.5]dec-2-ene (Compound No. 14);
3-[3-(2,3-Dihydro-1*H*-inden-2-yloxy)-4-methoxyphenyl]-1-oxa-2-azaspiro[4.5]dec-2-ene (Compound No. 15);
7-[3-(2,3-Dihydro-1H inden-2-yloxy)-4-isopropoxyphenyl]-5-oxa-6-azaspiro[3.4]oct-6-ene (Compound No. 16);
3-[4-Butoxy-3-(2,3-dihydro-1H inden-2-yloxy)phenyl]-1-oxa-2-azaspiro[4.4]non-2-ene (Compound No. 17);
3-[4-(Cyclopropylmethoxy)-3-(2,3-dihydro-1*H*-inden-2-yloxy)phenyl]-1-oxa-2-azaspiro[4.4]non-2-ene (Compound No. 18);
7-[3-(2,3-Dihydro-1*H*-inden-2-yloxy)-4-propoxyphenyl]-5-oxa-6-azaspiro[3.4]oct-6-ene (Compound No. 19);
7-[4-butoxy-3-(2,3-dihydro-1*H*-inden-2-yloxy)phenyl]-5-oxa-6-azaspiro[3.4]oct-6-ene (Compound No. 20);
3-[2-(2,3-Dihydro-1*H* inden-2-yloxy)-4-(1-oxa-2-azaspiro[4.4]non-2-en-3-yl)phenoxy]propan-1-ol (Compound No. 21);
3-[3-(2,3-Dihydro-1*H*-inden-2-yloxy)-4-(2-morpholin-4-ylethoxy)phenyl]-1-oxa-2-azaspiro[4.4]non-2-ene (Compound No. 22);
[2-(2,3-Dihydro-1*H*-inden-2-yloxy)-4-(1,7-dioxa-2-azaspiro[4.4]non-2-en-3-yl)phenoxy]ethanol (Compound No. 23)
3-[3-(2,3-Dihydro-1*H* inden-2-yloxy)-4-methoxyphenyl]-1-oxa-2-azaspiro[4.4]non-2-ene (Compound No. 24);
7-[4-(Difluoromethoxy)-3-(2,3-dihydro-1*H*-inden-2-yloxy)phenyl]-5-oxa-6-azaspiro[3.4]oct-6-ene (Compound No. 25);
Ethyl [2-methoxy-5-(1-oxa-2-azaspiro[4.5]dec-2-en-3-yl)phenoxy]acetate (Compound No. 26);
2-[2-Methoxy-5-(1-oxa-2-azaspiro[4.5]dec-2-en-3-yl)phenoxy]-*N*-methylacetamide (Compound No. 27);
Ethyl [2-methoxy-5-(1-oxa-2-azaspiro[4.4]non-2-en-3-yl)phenoxy]acetate (Compound No. 28);
[2-Methoxy-5-(1-oxa-2-azaspiro[4.4]non-2-en-3-yl)phenoxy]acetic acid (Compound No. 29);
2-[2-Methoxy-5-(1-oxa-2-azaspiro[4.4]non-2-en-3-yl)phenoxy]acetamide (Compound No. 30);
2-[2-Methoxy-5-(1-oxa-2-azaspiro[4.4]non-2-en-3-yl)phenoxy]-*N*-methylacetamide (Compound No. 31);
*N*-Cyclopentyl-2-[2-methoxy-5-(1-oxa-2-azaspiro[4.4]non-2-en-3-yl)phenoxy]acetamide (Compound No. 32);
Ethyl [2-methoxy-5-(5-oxa-6-azaspiro[3.4]oct-6-en-7-yl)phenoxy]acetate (Compound No. 33);
[2-Methoxy-5-(5-oxa-6-azaspiro[3.4]oct-6-en-7-yl)phenoxy]acetic acid (Compound No. 34);
2-[2-Methoxy-5-(5-oxa-6-azaspiro[3.4]oct-6-en-7-yl)phenoxy]acetamide (Compound No. 35);
2-[2-Methoxy-5-(5-oxa-6-azaspiro[3.4]oct-6-en-7-yl)phenoxy]-N-methylacetamide (Compound No. 36);
2-[2-Methoxy-5-(1-oxa-2-azaspiro[4.5]dec-2-en-3-yl)phenoxy]acetamide (Compound No. 37);
[5-(1,7-Dioxa-2-azaspiro[4.4]non-2-en-3-yl)-2-methoxyphenoxy]acetic acid (Compound No. 3 8);
*Tert-butyl* {3-[2-methoxy-5-(5-oxa-6-azaspiro[3.4]oct-6-en-7-yl)phenoxy]propyl} carbamate (Compound No. 39);
*Tert-butyl* {3 -[2-methoxy-5-(1-oxa-2-azaspiro[4.4]non-2-en-3 -yl)phenoxy]propyl} carbamate (Compound No. 40);
*Tert-*butyl {3 -[2-methoxy-5-(1-oxa-2-azaspiro[4.5] dec-2-en-3 -yl)phenoxy]propyl} carbamate (Compound No. 41);
Methyl 5-[2-methoxy-5-(5-oxa-6-azaspiro[3.4]oct-6-en-7-yl)phenoxy]pentanoate (Compound No. 42);
Methyl 5-[2-methoxy-5-(1-oxa-2-azaspiro[4.5]dec-2-en-3-yl)phenoxy]pentanoate (Compound No. 43);
Methyl 5-[2-methoxy-5-(1-oxa-2-azaspiro[4.4]non-2-en-3-yl)phenoxy]pentanoate (Compound No. 44);
3-[3-({4-[(Benzyloxy)methyl] cyclohexyl}methoxy)-4-methoxyphenyl]-1,7-dioxa-2-azaspiro[4.4]non-2-ene (Compound No. 45)
*Tert*-butyl 4-{[2-methoxy-5-(1-oxa-2-azaspiro[4.4]non-2-en-3-yl)phenoxy]methyl}piperidine-1-carboxylate (Compound No. 46)
3-[2-Methoxy-5-(1-oxa-2-azaspiro[4.5]dec-2-en-3-yl)phenoxy]propan-1-ol (Compound No. 47);
3-[5-(1,7-Dioxa-2-azaspiro[4.4]non-2-en-3-yl)-2-methoxyphenoxy]propan-1-ol (Compound No. 48);
5-(1,8-Dioxa-2-azaspiro[4.5]dec-2-en-3-yl)-2-methoxyphenyl methanesulfonate (Compound No. 49);
2-Methoxy-5-(1-oxa-2-azaspiro[4.5]dec-2-en-3-yl)phenyl methanesulfonate (Compound No. 50);
Hydrochloride salt of 3-[2-Methoxy-5-(1-oxa-2-aza-spiro[4.5]dec-2-en-3-yl)-phenoxy]-propylamine (Compound No. 51)
(S)-3-[3-(Indan-2-yloxy)-4-methoxy-phenyl]-1,7-dioxa-2-aza-spiro[4.4]non-2-ene (Compound No. 52);
(R)-3-[3-(Indan-2-yloxy)-4-methoxy-phenyl]-1,7-dioxa-2-aza-spiro[4.4]non-2-ene (Compound No. 53);
3-[3-(2,3-Dihydro-1*H*-inden-1-yloxy)-4-methoxyphenyl]-1-oxa-2-azaspiro[4.5]dec-2-ene (Compound No. 54);
3-[3-(2,3-Dihydro-1*H*-inden-1-yloxy)-4-methoxyphenyl]-1-oxa-2-azaspiro[4.4]non-2-ene (Compound No. 55);
7-[3-(2,3-Dihydro-1*H*-inden-1-yloxy)-4-methoxyphenyl]-5-oxa-6-azaspiro[3.4]oct-6-ene (Compound No. 56);
3-[3-(Benzyloxy)-4-methoxyphenyl]-1-oxa-2-azaspiro[4.5]dec-2-ene (Compound No. 57);
2-[2-Methoxy-5-(1-oxa-2-azaspiro[4.4]non-2-en-3-yl)phenoxy]-*N,N*-dimethylacetamide (Compound No. 58).

In another aspect, provided are pharmaceutical compositions comprising a therapeutically effective amount of a compound described herein.

In another aspect, provided are methods of treating AIDS, asthma, arthritis, bronchitis, chronic obstructive pulmonary disease (COPD), psoriasis, allergic rhinitis, shock, atopic dermatitis, Crohn's disease, adult respiratory distress syndrome (ARDS), eosinophilic granuloma, allergic conjunctivitis, osteoarthritis or ulcerative colitis comprising administering to an animal or human in need thereof a therapeutically effective amount of a compound described herein.

In another aspect, provided are methods of preventing, inhibiting or suppressing an inflammatory condition comprising administering to an animal or human in need thereof a therapeutically effective amount of a compound described herein.

In another aspect, provided are methods for preparing a compound of Formula VII, VIII, IX, X or Xa, or its pharmaceutically acceptable salts, pharmaceutically acceptable solvates, enantiomers, diastereomers or N-oxides comprising the steps of:
a. reacting a compound of Formula II 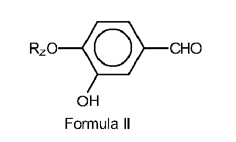 with a compound of Formula III 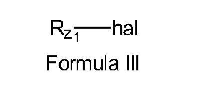 to form a compound of Formula IV, 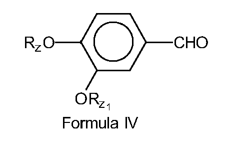
b. reacting the compound of Formula IV with hydroxylamine hydrochloride to form a compound of Formula V, and 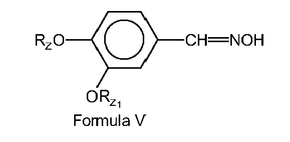
c. reacting the compound of Formula V with a compound of Formula VI 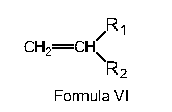 to form a compound of Formula VII, 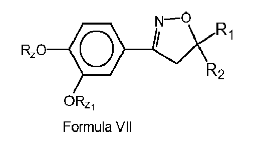
d. optionally hydrolyzing the compound of Formula VII (when Rz1 is -CH₂COOalkyl) to form a compound of Formula VIII, 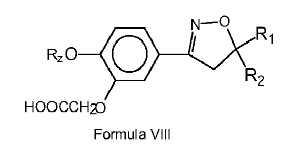
e. optionally reacting the compound of Formula VIII with a compound of Formula -NHRₓR_{y} to form a compound of Formula IX, 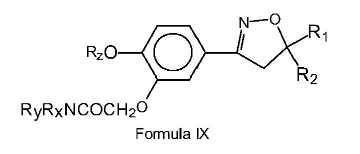
f. optionally reacting the compound of Formula VII with methanolic ammonia to form a compound of Formula X, 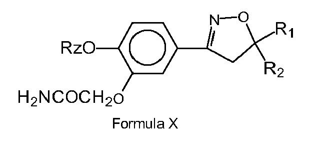
g. optionally deprotecting the compound of Formula VII (when -(CH₂)g1NHCOOalkyl) to form a compound of Formula Xa 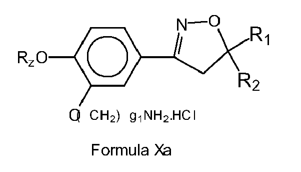
wherein
hal can be Cl, Br or I;
R_{z} can be alkyl optionally substituted with halogen or alkaryl;
R_{z1} can be alkyl, cycloalkyl, alkaryl, alkenyl, cycloalkylalkyl, heteroaryl, heterocyclyl, heteroarylalkyl, heterocyclylalkyl or -(CH₂)_{g1}C(=O)OR₃;
R₁ and R₂ can together form an optionally substituted cycloalkyl or heterocyclyl ring wherein the optional substituent is oxo, alkyl, alkenyl, alkynyl, halogen, nitro, -NH₂, -C(=O)NRₓR_{y}, -NHCOOR₆, cyano, hydroxy, alkoxy, or substituted amino;
R₃ can be alkyl, cycloalkyl or heterocyclyl;
g₁ can be an integer from 1-3;
Rₓ and R_{y} each independently can be hydrogen, alkyl, C₃-C₆ alkenyl, C₃-C₆ alkynyl, carboxy, cycloalkyl, -S(O)ₘR₅, aryl, alkaryl, heteroaryl, heterocyclyl, heteroarylalkyl or heterocyclylalkyl;
m can be an integer between 0-2;
R₆ can be alkyl, alkenyl, alkynyl, cycloalkyl, alkaryl, heteroarylalkyl or heterocyclylalkyl; and
R₅ can be hydrogen, alkyl, alkenyl, alkynyl, aryl, cycloalkyl, alkaryl, heteroaryl, heteroarylalkyl, heterocyclyl or heterocyclylalkyl.

In another aspect, provided are methods for preparing a compound of Formula XII or XIII, or its pharmaceutically acceptable salts, pharmaceutically acceptable solvates, enantiomers, diastereomers or N-oxides, comprising the steps of:
a. demethylating a compound of Formula XI 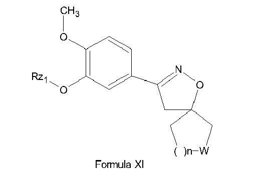 to form a compound of Formula XII, and 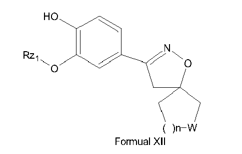
b. optionally reacting the compound of Formula XII with a compound of Formula C'-hal to form a compound of Formula XIII, 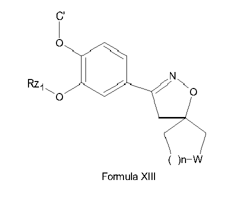
wherein
C' can be heterocyclylalkyl, cycloalkylalkyl, hydroxyalkyl, cycloalkyl or C₂₋₁₀ alkyl optionally substituted with halogen;
hal can be Cl, Br or I;
R_{z1} can be alkyl, cycloalkyl, alkaryl, alkenyl, cycloalkylalkyl, heteroaryl, heterocyclyl, heteroarylalkyl, heterocyclylalkyl or -(CH₂)_{g1}C(=O)OR₃;
R₃ can be alkyl, cycloalkyl or heterocyclyl;
g₁ can be an integer from 1-3;
n can be 0-3; and
W can be oxygen or carbon.

In another aspect, provided are methods for preparing a compound of Formula XVI, or its pharmaceutically acceptable salts, pharmaceutically acceptable solvates, enantiomers, diastereomers or N-oxides, comprising the steps of:
a. reacting a compound of Formula XIV 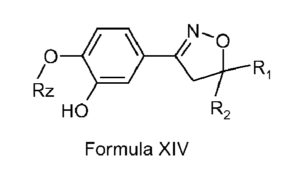 with a compound of Formula XV

   Rw-G Formula XV

   to form a compound of Formula XVI, 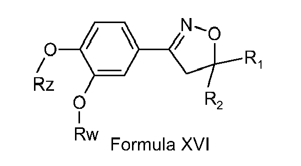
wherein
R_{w} can be alkyl, cycloalkyl, heteroaryl, heterocyclyl or -SO₂R₅;
G can be hal (wherein hal can be Cl, Br or I) or -OH;
R_{z} can be alkyl optionally substituted with halogen or alkaryl;
R₁ and R₂ can together form an optionally substituted cycloalkyl or heterocyclyl ring, wherein the optional substituent is oxo, alkyl, alkenyl, alkynyl, halogen, nitro, -NH₂, -C(=O)NRₓR_{y}, -NHCOOR₆, cyano, hydroxy, alkoxy, or substituted amino;
R₃ can be alkyl, cycloalkyl or heterocyclyl;
g₁ can be an integer from 1-3;
Rₓ and R_{y} each independently can be hydrogen, alkyl, C₃-C₆ alkenyl, C₃-C₆ alkynyl, carboxy, cycloalkyl, -S(O)ₘR₅, aryl, alkaryl, heteroaryl, heterocyclyl, heteroarylalkyl or heterocyclylalkyl;
m can be an integer between 0-2;
R₆ can be alkyl, alkenyl, alkynyl, cycloalkyl, alkaryl, heteroarylalkyl or heterocyclylalkyl; and
R₅ can be hydrogen, alkyl, alkenyl, alkynyl, aryl, cycloalkyl, alkaryl, heteroaryl, heteroarylalkyl, heterocyclyl or heterocyclylalkyl.

In another aspect, provided are methods for preparing a compound of Formula XXI, or its pharmaceutically acceptable salts, pharmaceutically acceptable solvates, enantiomers, diastereomers or N-oxides, comprising the steps of:
a. reacting a compound of Formula XVII 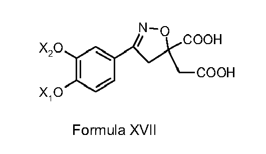 with a compound of Formula XVIIA

   Q Formula XVIIA

   to form a compound of Formula XVIII, 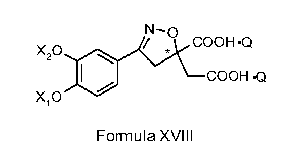
b. reacting the compound of Formula XVIII with a compound of Formula P'-OH to form a compound of Formula XIX, 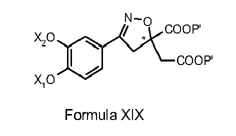
c. reducing the compound of Formula XIX to form a compound of Formula XX, and 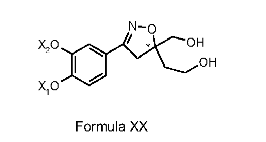
d. cyclizing the compound of Formula XX to form a compound of Formula XXI, 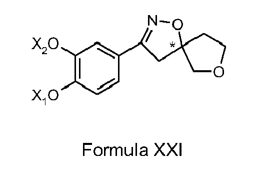
wherein
X₁ and X₂ can be hydrogen, alkyl, cycloalkyl, alkaryl, cycloalkylalkyl, heteroaryl, heterocyclyl, heteroarylalkyl, heterocyclylalkyl, -SO₂R₅, -(CH₂)_{g}NHCOOalkyl, - (CH₂)_{g}C(=O)NRₓR_{y} or -(CH₂)_{g1}C(=O)OR₃ (wherein g can be an integer from 0-3 and g₁ is an integer from 1-3);
X₁ and X₂ can together optionally form a cyclic ring fused with ring A of Formula I, wherein ring A contains 3-5 carbon atoms and 2-3 heteroatoms selected from N, O or S;
Q can be a chiral resolving agent; and
P' can be alkyl.

### Detailed Description of the Invention

The present invention provides compounds having a structure of Formula I, 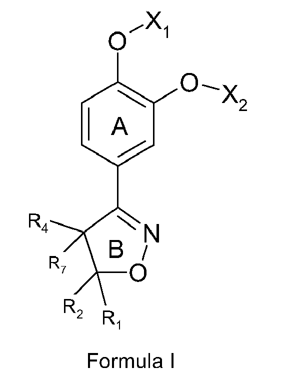 and its pharmaceutically acceptable salts, pharmaceutically acceptable solvates, enantiomers, diastereomers or N-oxides, wherein
- R₁ and R₂: together can form an optionally substituted cycloalkyl or heterocyclyl ring, wherein the optional substituent can be oxo, alkyl, alkenyl, alkynyl, halogen, nitro, -NH₂, -C(=O)NRₓR_{y}, -NHCOOR₆, cyano, hydroxy, alkoxy, or substituted amino;
- R₄: can be hydrogen, alkyl, hydroxy, halogen or carboxy;
- R₇: can be hydrogen or alkyl;
- X₁ and X₂: can be hydrogen, alkyl, cycloalkyl, alkaryl, cycloalkylalkyl, heteroaryl, heterocyclyl, heteroarylalkyl, heterocyclylalkyl, -SO₂R₅, -(CH₂)_{g}NHCOOalkyl, -(CH₂)_{g}C(=O)NRₓR_{y} or - (CH₂)_{g1}C(=O)OR₃ (wherein g can be an integer from 0-3 and g₁ can be an integer from 1-3);
- X₁ and X₂: together can optionally form a cyclic ring fused with ring A shown in Formula I, wherein ring A contains 3-5 carbon atoms and 2-3 heteroatoms selected from N, O or S;
wherein
R₃ can be alkyl, cycloalkyl or heterocyclyl;
halogen can be F, Cl, Br, or I;
Rₓ and R_{y} each independently can be hydrogen, alkyl, C₃-C₆ alkenyl, C₃-C₆ alkynyl, carboxy, cycloalkyl, -S(O)ₘR₅, aryl, alkaryl, heteroaryl, heterocyclyl, heteroarylalkyl, and heterocyclylalkyl;
m can be an integer between 0-2;
R₆ can be alkyl, alkenyl, alkynyl, cycloalkyl, alkaryl, heteroarylalkyl or heterocyclylalkyl; and
R₅ can be hydrogen, alkyl, alkenyl, alkynyl, aryl, cycloalkyl, alkaryl, heteroaryl, heteroarylalkyl, heterocyclyl or heterocyclylalkyl.

The following definitions apply to terms as used herein:

The term "alkyl," unless otherwise specified, refers to a monoradical branched or unbranched saturated hydrocarbon having from 1 to about 20 carbon atoms. This term is exemplified by groups, such as methyl, ethyl, n-propyl, iso-propyl, n-butyl, iso-butyl, t-butyl, n-hexyl, n-decyl, tetradecyl, and the like. The alkyl groups may be further substituted with one or more substituents such as alkenyl, alkynyl, alkoxy, cycloalkyl, acyl, acylamino, acyloxy, amino, aminocarbonyl, alkoxycarbonylamino, azido, cyano, halogen, hydroxy, oxo, thiocarbonyl, carboxy, arylthio, thiol, alkylthio, aryloxy, aminosulfonyl, aminocarbonylamino, hydroxyamino, alkoxyamino, nitro, -S(O)ₘR₅ (wherein m and R₅ are the same as defined earlier), heterocyclyl or heteroaryl. Unless otherwise constrained by the definition, all substituents may optionally be further substituted by 1-3 substituents chosen from alkyl, carboxy, aminocarbonyl, hydroxy, alkoxy, halogen, -CF₃, amino, substituted amino, cyano, and -S(O)ₘR₅ (wherein m and R₅ are the same as defined earlier) or an alkyl group as defined above that is interrupted by 1-5 atoms or groups independently chosen from oxygen, sulfur and -NRₐ- (where Rₐ can be hydrogen, alkyl, cycloalkyl, alkenyl, alkynyl, or aryl). Unless otherwise constrained by the definition, all substituents may optionally be further substituted by 1-3 substituents chosen from alkyl, carboxy, aminocarbonyl, hydroxy, alkoxy, halogen, CF₃, amino, substituted amino, cyano, and -S(O)ₘR₅ (wherein m and R₅ are the same as defined earlier); or an alkyl group as defined above that has both substituents as defined above and is also interrupted by 1-5 atoms or groups as defined above.

The term "alkenyl," unless otherwise specified, refers to a monoradical of a branched or unbranched unsaturated hydrocarbon group preferably having from 2 to 20 carbon atoms with cis or trans geometry. Preferred alkenyl groups include ethenyl or vinyl (CH=CH₂), 1-propylene or allyl (-CH₂CH=CH₂), or iso-propylene (-C(CH₃)=CH₂), bicyclo[2.2.1]heptene, and the like. In the event that the alkenyl is attached to a heteroatom, the double bond cannot be alpha to the heteroatom. The alkenyl group may be further substituted with one or more substituents, such as alkyl, alkenyl, alkynyl, alkoxy, cycloalkyl, acyl, acylamino, acyloxy, amino, aminocarbonyl, alkoxycarbonylamino, azido, cyano, halogen, hydroxy, oxo, thiocarbonyl, carboxy, arylthio, thiol, alkylthio, aryl, aryloxy, aminosulfonyl, aminocarbonylamino, hydroxyamino, alkoxyamino, nitro, -S(O)ₘR₅ (wherein m and R₅ are the same as defined earlier), heterocyclyl or heteroaryl. Unless otherwise constrained by the definition, all substituents may be optionally further substituted by 1-3 substituents, which can be alkyl, carboxy, aminocarbonyl, hydroxy, alkoxy, halogen, -CF₃, amino, substituted amino, cyano, or -S(O)ₘR₅ (wherein R₅ and m are the same as defined earlier).

The term "alkynyl," unless otherwise specified, refers to a monoradical of an unsaturated hydrocarbon, preferably having from 2 to 20 carbon atoms. Preferred alkynyl groups include ethynyl, (-C=CH), or propargyl (or propynyl, -CH₂C=CH), and the like. In the event that the alkynyl is attached to a heteroatom, the triple bond cannot be alpha to the heteroatom. The alkynyl group may be further substituted with one or more substituents, such as alkyl, alkenyl, alkynyl, alkoxy, cycloalkyl, acyl, acylamino, acyloxy, amino, aminocarbonyl, alkoxycarbonylamino, azido, cyano, halogen, hydroxy, oxo, thiocarbonyl, carboxy, arylthio, thiol, alkylthio, aryl, aryloxy, aminosulfonyl, aminocarbonylamino, hydroxyamino, alkoxyamino, nitro, or -S(O)ₘR₅ (wherein m and R₅ are the same as defined earlier). Unless otherwise constrained by the definition, all substituents may be optionally further substituted by 1-3 substituents, which can be alkyl, carboxy, aminocarbonyl, hydroxy, alkoxy, halogen, CF₃, amino, substituted amino, cyano or -S(O)ₘR₅ (wherein R₅ and m are the same as defined earlier).

The term "cycloalkyl," unless otherwise specified, refers to saturated or unsaturated cyclic alkyl groups of from 3 to 20 carbon atoms having a single cyclic ring or multiple condensed rings, which contains an optional olefinic bond. Such cycloalkyl groups include, by way of example, single ring structures, such as cyclopropyl, cyclobutyl, cyclopentyl, cyclooctyl, cyclopropylene, cyclobutylene and the like, or multiple ring structures, such as adamantanyl, and bicyclo [2.2.1]heptane, or cyclic alkyl groups to which is fused an aryl group, for example, indane and the like. The cycloalkyl may be further substituted with one or more substituents such as alkyl, alkenyl, alkynyl, alkoxy, cycloalkyl, acyl, acylamino, acyloxy, amino, aminocarbonyl, alkoxycarbonylamino, azido, cyano, halogen, hydroxy, oxo, thiocarbonyl, carboxy, arylthio, thiol, alkylthio, aryl, aryloxy, alkaryloxy, aminosulfonyl, aminocarbonylamino, hydroxyamino, alkoxyamino, nitro, -S(O)ₘR₅ (wherein m and R₅ are the same as defined earlier), heteroaryl or heterocyclyl. Unless otherwise constrained by the definition, all substituents may be optionally further substituted by 1-3 substituents, which can be alkyl, carboxy, aminocarbonyl, hydroxy, alkoxy, halogen, CF₃, -NH₂, substituted amino, cyano, or -S(O)ₘR₅ (wherein R₅ and m are the same as defined earlier).

The term "alkoxy" denotes the group O-alkyl, wherein alkyl is the same as defined above.

The term "alkaryl" refers to alkyl-aryl linked through alkyl portion (wherein alkyl is the same as defined earlier) and the alkyl portion contains carbon atoms from 1-6 and aryl is same as defined below.

The term "aryl," unless otherwise specified, refers to phenyl or naphthyl ring, and the like, optionally substituted with 1 to 3 substituents selected from the group consisting of halogen (such as F, Cl, Br, I), hydroxy, alkyl, alkenyl, alkynyl, cycloalkyl, alkoxy, aryloxy, -S(O)ₘR₅ (wherein R₅ is the same as defined earlier), cyano, nitro, carboxy, heterocyclyl, heteroaryl, heterocyclylalkyl, heteroarylalkyl, acyl and (CH₂)₀₋₃C(=O)NRₓR_{y} (wherein Rₓ and R_{y} are same as defined earlier).

The term "carboxy," unless otherwise specified, refers to -C(=O)O-R₆, wherein R₆ can be, for example, hydrogen, alkyl, alkenyl, alkynyl, cycloalkyl, alkaryl, heteroarylalkyl or heterocyclylalkyl.

The term "heteroaryl," unless otherwise specified, refers to an aromatic ring structure containing 5 or 6 carbon atoms, or a bicyclic aromatic group having 8 to 10 carbon atoms, with one or more heteroatom(s) independently selected from the group consisting of N, O and S, optionally substituted with 1 to 3 substituent(s), such as halogen (F, Cl, Br, I), hydroxy, alkyl, alkenyl, alkynyl, cycloalkyl, aryl, -S(O)ₘR₅ (wherein m and R₅ are the same as defined earlier), alkoxy, alkaryl, cyano, nitro, acyl or C(=O)NRₓR_{y} (wherein Rₓ and R_{y} are the same as defined earlier). Examples of heteroaryl groups include, but are not limited to, pyridinyl, pyridazinyl, pyrimidinyl, pyrrolyl, oxazolyl, thiazolyl, thienyl, isoxazolyl, triazinyl, furanyl, benzofuranyl, indolyl, benzothiazolyl, benzoxazolyl, and the like, including analogous oxygen, sulphur, and mixed hetero atom containing groups.

The term "heterocyclyl," unless otherwise specified, refers to a saturated or unsaturated monocyclic or polycyclic ring having 5 to 10 atoms, in which 1 to 3 carbon atoms in a ring are replaced by heteroatoms selected from the group consisting of O, S and N, and optionally are benzofused or fused heteroaryl of 5-6 ring members and/or optionally are substituted, wherein the substituents can be halogen (F, Cl, Br, I), hydroxy, alkyl, alkenyl, alkynyl, hydroxyalkyl, cycloalkyl, carboxy, aryl, alkoxy, alkaryl, heteroaryl, heterocyclyl, heteroarylalkyl, heterocyclylalkyl, oxo, alkoxyalkyl or -S(O)ₘR₅ (wherein m and R₅ are the same as defined earlier), cyano, nitro, -NH₂ substituted amino, acyl or -C(=O)NRₓR_{y} (wherein Rₓ and R_{y} are the same as defined earlier). Examples of heterocyclyl groups include, but are not limited to, tetrahydrofuranyl, dihydrofuranyl, azabicyclohexane dihydropyridinyl, piperidinyl, isoxazoline, piperazinyl, dihydrobenzofuryl, isoindole-dione, dihydroindolyl, and the like.

"Heteroarylalkyl," unless otherwise specified, refers to an alkyl-heteroaryl group, wherein the alkyl and heteroaryl portions are the same as defined earlier.

"Heterocyclylalkyl," unless otherwise specified, refers to an alkyl-heterocyclyl group, wherein the alkyl and heterocyclyl portions of the group are the same as defined earlier.

The term "acyl" as defined herein refers to -C(=O)R", wherein R" is the same as defined earlier.

The term "substituted amino," unless otherwise specified, refers to a group -N(Rₖ)₂ wherein each Rₖ can be hydrogen [provided that both Rₖ groups are not hydrogen (defined as "-NH₂")], alkyl, alkenyl, alkynyl, alkaryl, cycloalkyl, aryl, heteroaryl, heterocyclyl, heterocyclylalkyl, heteroarylalkyl, acyl, S(O)ₘR₅ (wherein m and R₅ is the same as defined above), -C(=O)NRₓR_{y}, -C(=O)ORₓ (wherein Rₓ and R_{y} are the same as defined earlier) or -NHC(=O)NR_{y}Rₓ (wherein R_{y} and Rₓ are the same as defined earlier).

Unless otherwise constrained by the definition, all substituents optionally may be further substituted by 1-3 substituents, which can be alkyl, alkaryl, cycloalkyl, aryl, heteroaryl, heterocyclyl, carboxy, hydroxy, alkoxy, halogen, -CF₃, cyano, -C(=O)NRₓR_{y}, -O(C=O)NRₓR_{y} (wherein Rₓ and R_{y} are the same as defined earlier) and -OC(=O)NRₓR_{y} or -S(O)ₘR₅ (where R₅ is the same as defined above and m is 0, 1 or 2).

The compounds described herein can be used for treating AIDS, asthma, arthritis, bronchitis, chronic obstructive pulmonary disease, psoriasis, allergic rhinitis, shock, atopic dermatitis, Crohn's disease, adult respiratory distress syndrome, eosinophilic granuloma, allergic conjunctivitis, osteoarthritis, ulcerative colitis and other inflammatory diseases. Accordingly, the present invention provides methods of treating AIDS, asthma, arthritis, bronchitis, chronic obstructive pulmonary disease, psoriasis, allergic rhinitis, shock, atopic dermatitis, Crohn's disease, adult respiratory distress syndrome, eosinophilic granuloma, allergic conjunctivitis, osteoarthritis, ulcerative colitis or other inflammatory diseases, which comprises administering to a patient in need thereof a therapeutically effective amount of an isoxazoline derivative compound described herein, and particularly an isoxazoline derivative compound described herein together a pharmaceutically acceptable carrier, excipient or diluent.

In accordance with yet another aspect, there are provided processes for the preparation of the compounds as described herein.

The compounds described herein may be prepared by techniques well known in the art. In addition, the compounds described herein may be prepared following reaction sequences as depicted below.

The compounds described herein contain one or more asymmetric carbon atoms and thus occur as racemic mixtures, enantiomers and diastereomers. These compounds also exist as conformers/rotamers. All such isomeric forms of these compounds are expressly included in the present invention. Each stereogenic carbon may have an R or S configuration. Although the specific compounds exemplified in this application may be depicted in a particular stereochemical configuration, compounds having either the opposite stereochemistry at any given chiral center or mixtures thereof are encompassed herein.

The compounds described herein may be prepared by techniques well known in the organic synthesis and familiar to a practitioner skilled in art. In addition, processes described herein may prepare the compounds described herein. However, the preparation of compounds described herein is not limited by such processes. Further, the various synthetic steps described herein may be performed in any alternate sequence to form the compounds herein.

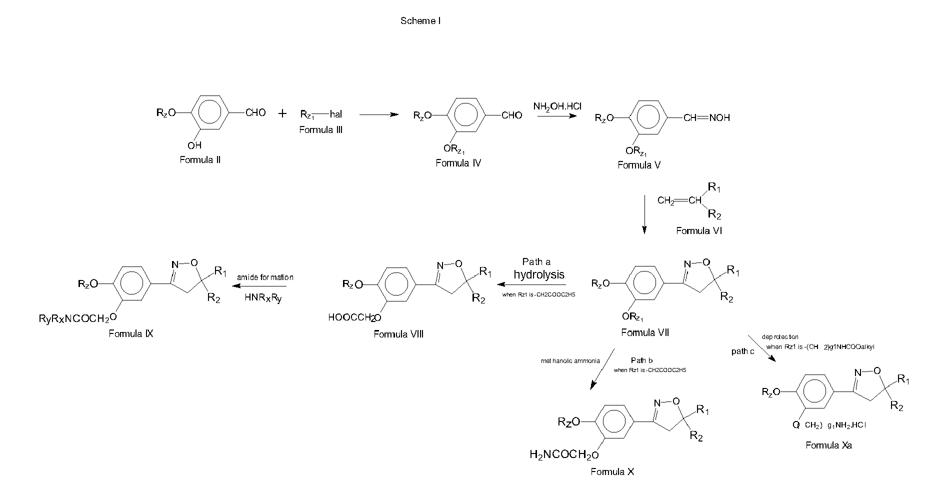

Compounds of Formulae VII and VIII, IX and X can be prepared by following the reaction sequence of Scheme I. Thus, compounds of Formula II (wherein R_{z} can be alkyl optionally substituted with halogen (for example, trifluoromethyl) or alkaryl (for example, benzyl)) can be reacted with compounds of Formula III (wherein hal can be Cl, Br or I and Rz1 can be alkyl, cycloalkyl, alkaryl, alkenyl, cycloalkylalkyl, heteroaryl, heterocyclyl, heteroarylalkyl, heterocyclylalkyl, -(CH₂)g₁NHCOOalkyl or -(CH₂)_{g1}C(=O)OR₃ wherein g1 and R₃ can be the same as defined earlier) to form compounds of Formula IV. Compounds of Formula IV can be reacted with hydroxylamine hydrochloride to form compounds of Formula V. Compounds of Formula V can be reacted with compounds of Formula VI (wherein R₁ and R₂ can be the same as defined earlier) to form compounds of Formula VII. Compounds of Formula VII can be reacted via three paths.

Path a: Compounds of Formula VII can be hydrolyzed (when Rz₁ is -CH₂COOalkyl) to form compounds of Formula VIII. Compounds of Formula VIII can be reacted with compounds of Formula -NHRₓR_{y} to form compounds of Formula IX.

Path b: Compounds of Formula VII can be reacted with methanolic ammonia to form compounds of Formula X.

Path c: Compounds of Formula VII (when Rz₁ is -(CH₂)g1NHCOOalkyl) can be deprotected to form compounds of Formula Xa.

Compounds of Formula II can be reacted with compounds of Formula III to form compounds of Formula IV in one or more organic solvents, for example, dimethylformamide, tetrahydrofuran, diethylether, dioxane or mixtures thereof. The reaction can also be carried out in the presence of one or more bases, for example, potassium carbonate, sodium carbonate, sodium bicarbonate or mixtures thereof.

Compounds of Formula IV can be reacted with hydroxylamine hydrochloride to form compounds of Formula V in one or more organic solvents, for example, ethanol, methanol, propanol or isopropyl alcohol.

Compounds of Formula V can be reacted with compounds of Formula VI to form compounds of Formula VII in one or more organic solvents, for example, tetrahydrofuran, dimethylformamide, dioxane or diethylether. This reaction can also be carried out in the presence of one or more oxidants, for example, sodium hypochlorite, N-chlorosuccinimide, tert-butoxychloride or mixtures thereof in the presence of one or more optional bases, for example, pyridine, butyl lithium, N-methylmorpholine, diisopropylethylamine, triethylamine or mixtures thereof.

Compounds of Formula VII (Path a, when Rz1 is -CH₂COOC₂H₅) can be hydrolyzed to form compounds of Formula VIII in one or more solvents, for example, tetrahydrofuran, methanol, dioxane or ethanol in water. The hydrolysis can also be carried out in the presence of one or more bases, for example, lithium hydroxide, sodium hydroxide, potassium hydroxide or mixtures thereof.

Compounds of Formula VIII can be reacted with compounds of Formula -NHRₓR_{y} to form compounds of Formula IX in one or more organic solvents, for example, dichloromethane, dichloroethane, chloroform, carbon tetrachloride or mixtures thereof The reaction can also be carried out in the presence of one or more halogenating agents, for example, thionyl chloride, phosphorous pentachloride, phosphorous trichloride or mixtures thereof.

Compounds of Formula VII can be reacted with methanolic ammonia to form compounds of Formula X.

Compounds of Formula VII (when Rz1 is -(CH₂)g1NHCOOalkyl) can be deprotected to form compounds of Formula Xa in the presence of one or more deprotecting agents selected from methanolic hydrochloric acid, ethanolic hydrochloric acid, trifluoroacetic acid, concentrated hydrochloric acid in ethyl acetate, tetrahydrofuran or mixtures thereof

The compounds prepared following Scheme I include, for example:
Ethyl [2-methoxy-5-(1-oxa-2-azaspiro[4.5]dec-2-en-3-yl)phenoxy]acetate (Compound No. 26);
2-[2-Methoxy-5-(1-oxa-2-azaspiro[4.5]dec-2-en-3-yl)phenoxy]-*N*-methylacetamide (Compound No. 27);
Ethyl [2-methoxy-5-(1-oxa-2-azaspiro[4.4]non-2-en-3-yl)phenoxy]acetate Compound No. 28);
[2-Methoxy-5-(1-oxa-2-azaspiro[4.4]non-2-en-3-yl)phenoxy]acetic acid (Compound No. 29);
2-[2-Methoxy-5-(1-oxa-2-azaspiro[4.4]non-2-en-3-yl)phenoxy]acetamide (Compound No. 30);
2-[2-Methoxy-5-(1-oxa-2-azaspiro[4.4]non-2-en-3-yl)phenoxy]-*N*-methylacetamide (Compound No. 31);
*N*-Cyclopentyl-2-[2-methoxy-5-(1-oxa-2-azaspiro[4.4]non-2-en-3-yl)phenoxy]acetamide (Compound No. 32);
Ethyl [2-methoxy-5-(5-oxa-6-azaspiro[3.4]oct-6-en-7-yl)phenoxy]acetate (Compound No. 33);
[2-Methoxy-5-(5-oxa-6-azaspiro[3.4]oct-6-en-7-yl)phenoxy]acetic acid (Compound No. 34);
2-[2-Methoxy-5-(5-oxa-6-azaspiro[3.4]oct-6-en-7-yl)phenoxy]acetamide (Compound No. 35);
2-[2-Methoxy-5-(5-oxa-6-azaspiro[3.4]oct-6-en-7-yl)phenoxy]-N-methylacetamide (Compound No. 36);
2-[2-Methoxy-5-(1-oxa-2-azaspiro[4.5]dec-2-en-3-yl)phenoxy]acetamide (Compound No. 37);
[5-(1,7-Dioxa-2-azaspiro[4.4]non-2-en-3-yl)-2-methoxyphenoxy]acetic acid (Compound No. 3 8);
*Tert*-butyl {3-[2-methoxy-5-(5-oxa-6-azaspiro[3.4]oct-6-en-7-yl)phenoxy]propyl} carbamate (Compound No. 39);
*Tert*-butyl {3-[2-methoxy-5-(1-oxa-2-azaspiro[4.4]non-2-en-3-yl)phenoxy]propyl} carbamate (Compound No. 40);
*Tert*-butyl {3-[2-methoxy-5-(1-oxa-2-azaspiro[4.5]dec-2-en-3-yl)phenoxy]propyl} carbamate (Compound No. 41);
Methyl 5-[2-methoxy-5-(5-oxa-6-azaspiro[3.4]oct-6-en-7-yl)phenoxy]pentanoate (Compound No. 42);
Hydrochloride salt of 3-[2-Methoxy-5-(1-oxa-2-aza-spiro[4.5]dec-2-en-3-yl)-phenoxy]-propylamine (Compound No. 51);
3-[3-(2,3-Dihydro-1*H-*inden-1-yloxy)-4-methoxyphenyl]-1-oxa-2-azaspiro[4.5]dec-2-ene (Compound No. 54);
3-[3-(2,3-Dihydro-1*H*-inden-1-yloxy)-4-methoxyphenyl]-1-oxa-2-azaspiro[4.4]non-2-ene (Compound No. 55);
7-[3-(2,3-Dihydro-1*H*-inden-1-yloxy)-4-methoxyphenyl]-5-oxa-6-azaspiro[3.4]oct-6-ene (Compound No. 56);
3-[3-(Benzyloxy)-4-methoxyphenyl]-1-oxa-2-azaspiro[4.5]dec-2-ene (Compound No. 57); or
2-[2-Methoxy-5-(1-oxa-2-azaspiro[4.4]non-2-en-3-yl)phenoxy]-*N,N*-dimethylacetamide (Compound No. 58);
and its pharmaceutically acceptable salts, pharmaceutically acceptable solvates, enantiomers, diastereomers or N-oxides.

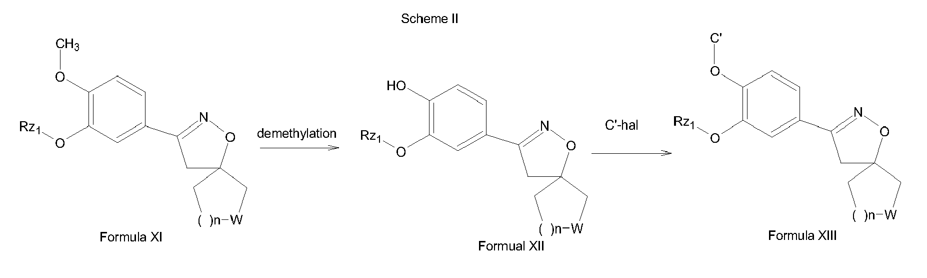

Compounds of Formulae XII and XIII can be prepared by following the procedure as depicted in Scheme II. Thus, compounds of Formula XI (which can be prepared following the procedure as described in, for example, WO 05/021515 and incorporated herein by reference) (wherein n can be 0-3, W can be oxygen or carbon, and Rz1 is the same as defined earlier) can be demethylated to form compounds of Formula XII. Compounds of Formula XII can be reacted with compounds of Formula C'-hal (wherein hal is the same as defined and C' can be heterocyclylalkyl, cycloalkylalkyl, hydroxyalkyl, cycloalkyl or C₂₋₁₀ alkyl optionally substituted with halogen) to form compounds of Formula XIII.

Compounds of Formula XI can be demethylated to form compounds of Formula XII in the presence of one or more reducing agents, for example, sodium ethane thiolate, sodium decane thiolate, sodium dodecane thiolate, sodium thiocresolate or mixtures thereof. The demethylation can also be carried out in one or more solvents, for example, N,N-dimethylacetamide, hexamethylphosphoramide, dimethylformamide or mixtures thereof

Compounds of Formula XII can be reacted with compounds of Formula C'-hal to form compounds of Formula XIII in one or more organic solvents, for example, dimethylformamide, tetrahydrofuran, diethyl ether, dioxane or mixtures thereof. The reaction can also be carried out in the presence of one or more bases, for example, potassium carbonate, sodium carbonate, lithium carbonate or mixtures thereof.

Compound prepared following Scheme II include, for example:
7-[3-(2,3-dihydro-1*H*-inden-2-yloxy)-4-methoxyphenyl]-5-oxa-6-azaspiro[3.4]oct-6-ene (Compound No. 1);
2-(2,3-dihydro-1*H*-inden-2-yloxy)-4-(5-oxa-6-azaspiro[3.4]oct-6-en-7-yl)phenol (Compound No. 2);
3-[3-(2,3-dihydro-1*H*-inden-2-yloxy)-4-propoxyphenyl]-1-oxa-2-azaspiro[4.4]non-2-ene (Compound No. 3);
3-[3-(2,3-dihydro-1*H*-inden-2-yloxy)-4-isopropoxyphenyl]-1-oxa-2-azaspiro[4.4]non-2-ene (Compound No. 4);
2-(2,3-dihydro-1*H*-inden-2-yloxy)-4-(1-oxa-2-azaspiro[4.4]non-2-en-3-yl)phenol (Compound No. 5);
3-[3-(2,3-Dihydro-1*H* inden-2-yloxy)-4-isobutoxyphenyl]-1-oxa-2-azaspiro[4.4]non-2-ene (Compound No. 6);
7-[3-(2,3 -Dihydro-1*H*-inden-2-yloxy)-4-ethoxyphenyl]-5-oxa-6-azaspiro[3.4]oct-6-ene (Compound No. 7);
2-(2,3-Dihydro-1*H*-inden-2-yloxy)-4-(1-oxa-2-azaspiro[4.5]dec-2-en-3-yl)phenol (Compound No. 8)
3-[4-Butoxy-3-(2,3-dihydro-1*H* inden-2-yloxy)phenyl]-1-oxa-2-azaspiro[4.5]dec-2-ene (Compound No. 9);
3-[3-(2,3-Dihydro-1*H*-inden-2-yloxy)-4-propoxyphenyl]-1-oxa-2-azaspiro[4.5]dec-2-ene (Compound No. 10);
3-[4-(Difluoromethoxy)-3-(2,3-dihydro-1*H*-inden-2-yloxy)phenyl]-1-oxa-2-azaspiro[4.5]dec-2-ene (Compound No. 11);
3 -[3-(2,3 -Dihydro-1*H*-inden-2-yloxy)-4-ethoxyphenyl]-1-oxa-2-azaspiro[4.5]dec-2-ene (Compound No. 12);
3-[4-(Cyclopropylmethoxy)-3-(2,3-dihydro-1*H*-inden-2-yloxy)phenyl]-1-oxa-2-azaspiro[4.5]dec-2-ene (Compound No. 13);
3-[3-(2,3-Dihydro-1*H*-inden-2-yloxy)-4-isopropoxyphenyl]-1-oxa-2-azaspiro[4.5]dec-2-ene (Compound No. 14);
3 -[3-(2,3 -Dihydro-1*H*-inden-2-yloxy)-4-methoxyphenyl]-1-oxa-2-azaspiro[4.5]dec-2-ene (Compound No. 15);
7-[3-(2,3 -Dihydro-1*H*-inden-2-yloxy)-4-isopropoxyphenyl]-5-oxa-6-azaspiro[3.4]oct-6-ene (Compound No. 16);
3-[4-Butoxy-3-(2,3-dihydro-1*H*-inden-2-yloxy)phenyl]-1-oxa-2-azaspiro[4.4]non-2-ene (Compound No. 17);
3-[4-(Cyclopropylmethoxy)-3-(2,3-dihydro-1*H*-inden-2-yloxy)phenyl]-1-oxa-2-azaspiro[4.4]non-2-ene (Compound No. 18);
7-[3-(2,3-Dihydro-1*H*-inden-2-yloxy)-4-propoxyphenyl]-5-oxa-6-azaspiro[3.4]oct-6-ene (Compound No. 19);
7-[4-butoxy-3-(2,3-dihydro-1*H* inden-2-yloxy)phenyl]-5-oxa-6-azaspiro[3.4]oct-6-ene (Compound No. 20);
3-[2-(2,3 -Dihydro-1*H*-inden-2-yloxy)-4-(1-oxa-2-azaspiro[4.4]non-2-en-3-yl)phenoxy]propan-1-ol (Compound No. 21);
3-[3-(2,3-Dihydro-1*H*-inden-2-yloxy)-4-(2-morpholin-4-ylethoxy)phenyl]-1-oxa-2-azaspiro[4.4]non-2-ene (Compound No. 22);
[2-(2,3-Dihydro-1*H*-inden-2-yloxy)-4-(1,7-dioxa-2-azaspiro[4.4]non-2-en-3-yl)phenoxy] ethanol (Compound No. 23)
3-[3-(2,3-Dihydro-1*H*-inden-2-yloxy)-4-methoxyphenyl]-1-oxa-2-azaspiro[4.4]non-2-ene (Compound No. 24);
7-[4-(Difluoromethoxy)-3-(2,3-dihydro-1*H*-inden-2-yloxy)phenyl]-5-oxa-6-azaspiro[3.4]oct-6-ene (Compound No. 25);
and its pharmaceutically acceptable salts, pharmaceutically acceptable solvates, enantiomers, diastereomers or N-oxides.

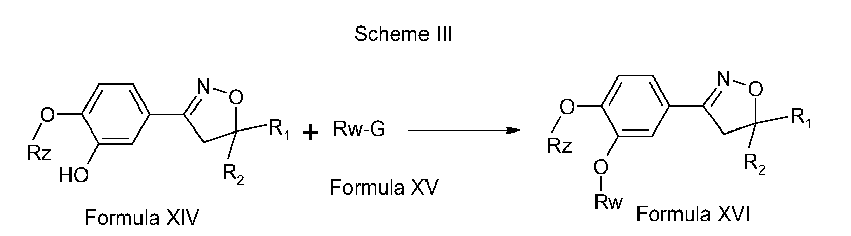

Compounds of Formula XVI can be prepared by following the reaction sequence as depicted in Scheme III. Thus, compounds of Formula XIV (prepared following the procedure reported in, for example, WO 05/021515, which is incorporated herein by reference) (wherein Rz is the same as defined above) can be reacted with compounds of Formula XV (wherein R_{w} can be alkyl, hydroxyalkyl, cycloalkyl, heteroaryl, heterocyclyl or -SO₂R₅ and G is -OH or hal (wherein hal is the same as defined earlier)) to form compounds of Formula XVI.

Compounds of Formula XIV can be reacted with compounds of Formula XV (when G is hal) to form compounds of Formula XVI in one or more organic solvents, for example, dimethylformamide, tetrahydrofuran, diethylether, dioxane or mixtures thereof The reaction can also be carried out in the presence of one or more bases, for example, potassium carbonate, sodium carbonate, sodium bicarbonate or mixtures thereof.

Compounds of Formula XIV can be reacted with compounds of Formula XV (when G is -OH) to form compounds of Formula XVI in one or more organic solvents, for example, tetrahydrofuran, diethylether, dioxane, toluene, benzene, dimethylformamide or mixtures thereof. The reaction can also be carried out in the presence of a redox couple. Suitable redox coupling agents include one or more oxidizing part and one or more reduction part, and may be any one of those known to a person skilled in the art of organic synthesis. The oxidizing part of the redox couple can be selected from one or more of diisopropylazodicarboxylate (DIAD), diethylazodicarboxylate (DEAD), N,N,N'N'-tetramethylazodicarboxamide (TMAD), 1,1'-(azodicarbonyl)dipiperidine (ADDP), cyanomethylenetributylphosphorane (CMBP), 4,7-dimethyl-3,5,7-hexahydro-1,2,4,7-tetrazocin-3,8-dione (DHTD), N,N,N'N'-tetraisopropylazodicarboxamide (TIPA) or mixtures thereof. The reduction part of the redox couple can be a phosphine selected from one or more oftrialkylphosphine (such as tributylphosphine), triarylphosphine (such as triphenylphosphine), tricycloalkylphosphine (such as tricyclohexylphosphine), triheteroarylphosphine or mixtures thereof. Phosphine reagents can include a combination of aryl, alkyl or heteroaryl substituents, for example, diphenylpyridylphosphine.

Compounds formed following Scheme III include, for example:
3-[3-({4-[(Benzyloxy)methyl]cyclohexyl}methoxy)-4-methoxyphenyl]-1,7-dioxa-2-azaspiro[4.4]non-2-ene (Compound No. 45);
*Tert-*butyl 4-{[2-methoxy-5-(1-oxa-2-azaspiro[4.4]non-2-en-3-yl)phenoxy]methyl}piperidine-1-carboxylate (Compound No. 46);
3-[2-Methoxy-5-(1-oxa-2-azaspiro[4.5]dec-2-en-3-yl)phenoxy]propan-1-ol (Compound No. 47);
3-[5-(1,7-Dioxa-2-azaspiro[4.4]non-2-en-3-yl)-2-methoxyphenoxy]propan-1-ol (Compound No. 48);
5-(1,8-Dioxa-2-azaspiro[4.5]dec-2-en-3-yl)-2-methoxyphenyl methanesulfonate (Compound No. 49);
2-Methoxy-5-(1-oxa-2-azaspiro[4.5]dec-2-en-3-yl)phenyl methanesulfonate (Compound No. 50);
Methyl 5-[2-methoxy-5-(1-oxa-2-azaspiro[4.5]dec-2-en-3-yl)phenoxy]pentanoate (Compound No. 43);
Methyl 5-[2-methoxy-5-(1-oxa-2-azaspiro[4.4]non-2-en-3-yl)phenoxy]pentanoate (Compound No. 44);
and its pharmaceutically acceptable salts, pharmaceutically acceptable solvates, enantiomers, diastereomers or N-oxides.

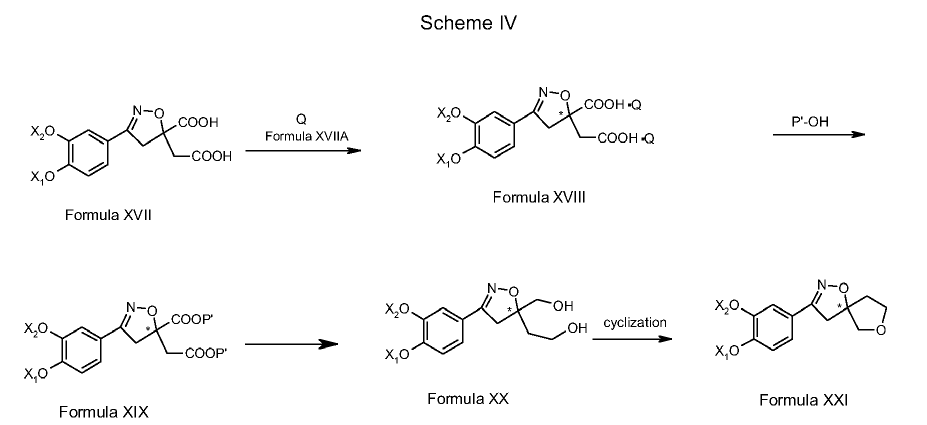

Compounds of Formula XXI can be prepared by following the procedure as depicted in Scheme IV. Thus, compounds of Formula XVII (wherein X₁ and X₂ are the same as defined earlier) can be reacted with compounds of Formula XVIIA (wherein Q can be a chiral resolving agent selected from L-Ephederine, D-Ephederine, Brucine, (1S, 2R) (-)-cis-1-amino-2-indanol, (1R 2S) (+)-cis-1-amino-2-indanol, (1R, 2R)-(-)-1,2-diamino cyclohexane or (1S, 2S)-(+)-1,2-diamino cyclohexane or α-methylbenzylamine) to form compounds of Formula XVIII. Compounds of Formula XVIII can be protected with compounds of Formula P'-OH to form compounds of Formula XIX (wherein P' can be alkyl). Compounds of Formula XIX can be reduced to form compounds of Formula XX. Compounds of Formula XX can be cyclized to form compounds of Formula XXI (wherein XXI represents S-isomer when L-Ephederine is used or R-isomer when D-Ephederine is used).

Compounds of Formula XVII can be reacted with compounds of Formula XVIIA to form compounds of Formula XVIII in one or more organic solvents, for example, acetone, dichloromethane, chloroform or mixtures thereof

Compounds of Formula XVIII can be protected by reacting with compounds of Formula P'-OH to form compounds of Formula XIX in the presence of one or more halogenating agents, for example, thionyl chloride, phosphorous pentachloride, phosphorous trichloride or mixtures thereof

Compounds of Formula XIX can be reduced to form compounds of Formula XX in one or more organic solvents, such as, for example, tetrahydrofuran, dimethylformamide, diethyl ether, dioxane or mixtures thereof. The reduction can be carried out in the presence of one or more reducing agents, for example, lithium aluminum hydride, sodium borohydride, lithium borohydride or mixtures thereof

Alternatively, compounds of Formula XX can also be prepared by reducing a free acid form of compounds of Formula XIX.

Compounds of Formula XX can be cyclized to form compounds of Formula XXI in one or more organic solvents, for example, tetrahydrofuran, dimethylformamide, dioxane, diethyl ether or mixtures thereof The cyclization reaction can be carried out in the presence of a redox couple. The oxidizing part of the redox couple can be selected from one or more of diisopropylazodicarboxylate (DIAD), diethylazodicarboxylate (DEAD), N,N,N',N'-tetramethylazodicarboxylate (TMAD), 1,1'-(azodicarbonyl) dipiperidine (ADDP), cyanomethylenetributylphosphorane (CMBP), 4,7-dimethyl-3,5,7-hexahydro-1,2,4,7-tetrazocin-3,8-dione (DHTD), N,N,N',N,'-tetraisopropylazodicarboxamide (TIPA) or mixtures thereof. The reduction part of the redox couple can be one or more phosphines selected from trialkylphosphine (such as tributylphosphine), triarylphosphine (such as triphenylphosphine), tricycloalkylphosphine (such as triscyclohexylphosphine), tetraheteroarylphosphine or mixtures thereof. Phosphine reagents can include a combination of aryl, alkyl or heteroaryl substituents, for example, diphenylpyridylphosphine.

Compounds prepared following Scheme IV include, for example:
(S)-3-[3-(Indan-2-yloxy)-4-methoxy-phenyl]-1,7-dioxa-2-aza-spiro[4.4]non-2-ene (Compound No. 52);
(R)-3-[3-(Indan-2-yloxy)-4-methoxy-phenyl]-1,7-dioxa-2-aza-spiro[4.4]non-2-ene (Compound No. 53).

In the above schemes, where specific reagents are described, including bases, condensing agents, hydrolyzing agents, solvents, etc., other similar reagents known to those skilled in the art may be used. Similarly, reaction conditions, for example, temperature and duration of the reaction, may be adjusted according to the desired needs.

While the present invention has been described in terms of its specific embodiments, certain modifications and equivalents will be apparent to those skilled in the art and are included within the scope of the present invention. The examples are provided to illustrate particular aspects of the disclosure and do not limit the scope of the present invention as defined by the claims.

### EXAMPLES

### Scheme I

### Example 1: General procedure for synthesis of compounds of Formula IX, X and Xa

### Step a: Synthesis of compounds of Formula IV

Potassium carbonate (10.91 g, 0.079 mol) was added to a solution of the compound of Formula II (6 g, 0.0395 mol) in dimethylformamide (100 mL) and the reaction mixture was heated to 60 °C. A compound of Formula III (0.0592 mol) was added to the reaction mixture and then stirred at 70-80 °C for 8 hours. The reaction mixture was filtered through celite, diluted with water and extracted with ethyl acetate. The organic extracts were collected, washed with brine, dried over anhydrous sodium sulphate and concentrated under reduced pressure. A residue thus obtained was purified by column chromatography to yield the title compound.

The following compounds were prepared by the above procedure using the appropriate corresponding reagents.
Ethyl (5-formyl-2-methoxyphenoxy)acetate
Methyl 5-(5-formyl-2-methoxyphenoxy)pentanoate
*Tert*-butyl [3-(5-formyl-2-methoxyphenoxy)propyl]carbamate

### Step b: Synthesis of compounds of Formula V

Hydroxylamine hydrochloride (1.69g, 0.0252 mol) and sodium acetate (2.07 g, 0.0252 mol) were added to a stirred solution of compound of Formula IV (0.12 mol) in ethanol (80 mL). The reaction mixture was stirred at room temperature overnight. Ethanol was evaporated under reduced pressure. The residue thus obtained was diluted with water (200 mL), extracted with dichloromethane, dried over anhydrous sodium sulphate, filtered and concentrated under reduced pressure to yield the title compound.

Following compounds were prepared by the above procedure using the appropriate corresponding reagents.
Ethyl {5-[(*E*)-(hydroxyimino)methyl]-2-methoxyphenoxy}acetate
Methyl 5-{5-[(*E*)-(hydroxyimino)methyl]-2-methoxyphenoxy}pentanoate
*Tert*-butyl (3-{5-[(*E*)-(hydroxyimino)methyl]-2-methoxyphenoxy}propyl)carbamate

### Step c: Synthesis of compounds of Formula VII

Pyridine (2-3 drops) and sodium hypochlorite solution (10 mL) were added dropwise to a solution of a compound of Formula V (0.00395 mol) and a compound of Formula VI (0.42 mL, 0.054 mol) in dichloromethane. The reaction mixture was stirred at room temperature for 5 hours. The organic layer was extracted with water twice and washed with saturated solution of sodium chloride. The organic layer was concentrated to yield the title compound.

Following compounds were prepared by the above procedure using the appropriate corresponding reagents.
Ethyl [2-methoxy-5-(1-oxa-2-azaspiro[4.5]dec-2-en-3-yl)phenoxy]acetate (Compound No. 26)
Mass (m/z): 348.3 (M⁺+1).
Ethyl [2-methoxy-5-(1-oxa-2-azaspiro[4.4]non-2-en-3-yl)phenoxy]acetate (Compound No. 28)
Mass (m/z): 334.1 (M⁺+1).
Ethyl [2-methoxy-5-(5-oxa-6-azaspiro[3.4]oct-6-en-7-yl)phenoxy]acetate (Compound No. 33) Mass (m/z): 320.2 (M⁺+1).
*Tert*-butyl {3-[2-methoxy-5-(5-oxa-6-azaspiro[3.4]oct-6-en-7-yl)phenoxy]propyl}carbamate (Compound No. 39)
Mass (m/z): 391.2 (M⁺+1).
*Tert*-butyl {3 -[2-methoxy-5-(1-oxa-2-azaspiro[4.4]non-2-en-3 -yl)phenoxy]propyl} carbamate (Compound No. 40)
Mass (m/z): 405.3 (M⁺+1).
*Tert*-butyl {3 -[2-methoxy-5-(1-oxa-2-azaspiro[4.5] dec-2-en-3 -yl)phenoxy]propyl} carbamate (Compound No. 41)
Mass (m/z): 419.3 (M⁺+1).
3-[3-(2,3-Dihydro-1*H*-inden-1-yloxy)-4-methoxyphenyl]-1-oxa-2-azaspiro[4.5]dec-2-ene (Compound No. 54)
Mass (m/z): 378.05 (M⁺+1).
3-[3-(2,3-Dihydro-1*H*-inden-1-yloxy)-4-methoxyphenyl]-1-oxa-2-azaspiro[4.4]non-2-ene (Compound No. 55)
Mass (m/z): 364.04 (M⁺+1).
7-[3-(2,3-Dihydro-1*H*-inden-1-yloxy)-4-methoxyphenyl]-5-oxa-6-azaspiro[3.4]oct-6-ene (Compound No. 56)
Mass (m/z): 350.05 (M⁺+1).
3-[3-(Benzyloxy)-4-methoxyphenyl]-1-oxa-2-azaspiro[4.5]dec-2-ene (Compound No. 57) Mass (m/z): 352.06 (M⁺+1).

### Step d: Synthesis of compounds of Formula VIII

A compound of Formula VII (0.0003 mol) was dissolved in tetrahydrofuran (20 mL) and lithium hydroxide in water solution (0.0006 mol) was added. The mixture was stirred at room temperature for 4 hours. Solvent was removed under reduced pressure and the residue thus obtained was diluted with water, acidified with concentrated hydrochloric acid. The organic compound was extracted with dichloromethane, washed with brine, dried over anhydrous sodium sulphate and concentrated under reduced pressure to yield title organic compound.

Following compounds were prepared by the above procedure using the appropriate corresponding reagents.
[2-Methoxy-5-(1-oxa-2-azaspiro[4.4]non-2-en-3-yl)phenoxy]acetic acid (Compound No. 29) Mass (m/z): 306.2 (M⁺+1).
[2-Methoxy-5-(5-oxa-6-azaspiro[3.4]oct-6-en-7-yl)phenoxy]acetic acid (Compound No. 34) Mass (m/z): 292.3 (M⁺+1).
[5-(1,7-Dioxa-2-azaspiro[4.4]non-2-en-3-yl)-2-methoxyphenoxy]acetic acid (Compound No. 38)
Mass (m/z): 308.2 (M⁺+1).

### Step e: Synthesis of compound of Formula IX

Thionyl chloride (0.033 mL, 0.00046 mol) was added slowly to a stirred solution of compound of Formula VIII (0.00023 mol) in dichloromethane (20 mL) at 0 °C. The reaction mixture was then stirred for an additional 4 hours at room temperature and under nitrogen atmosphere. A compound of Formula -NHRₓR_{y} (0.00092 mol) was added slowly to reaction mixture and stirred at room temperature overnight. The reaction mixture was diluted with water and extracted with dichloromethane. The organic layer was washed with brine and dried over anhydrous sodium sulphate and concentrated under reduced pressure to yield the title compound.

Following compounds were prepared by the above procedure using the appropriate corresponding reagents.
2-[2-Methoxy-5-(1-oxa-2-azaspiro[4.5]dec-2-en-3-yl)phenoxy]-*N*-methylacetamide (Compound No. 27)
Mass (m/z): 333.3 (M⁺+1).
2-[2-Methoxy-5-(1-oxa-2-azaspiro[4.4]non-2-en-3-yl)phenoxy]-N methylacetamide (Compound No. 31)
Mass (m/z): 319.2 (M⁺+1).
*N*-Cyclopentyl-2-[2-methoxy-5-(1-oxa-2-azaspiro [4.4]non-2-en-3-yl)phenoxy] acetamide (Compound No. 32)
Mass (m/z): 373.3 (M⁺+1).
2-[2-Methoxy-5-(5-oxa-6-azaspiro[3.4]oct-6-en-7-yl)phenoxy]-*N*-methylacetamide (Compound No. 36)
Mass (m/z): 305.4 (M⁺+1).

### Step f: Synthesis of compound of Formula X

A methanolic ammonia solution (10 mL) was added to a solution of a compound of Formula VII (0.00023 mol) and stirred for 3 hours at room temperature. The reaction mixture was concentrated under reduced pressure. The residue thus obtained was dissolved in dichloromethane (10 mL). The mixture was extracted with water, dried over anhydrous sodium sulphate and concentrated under reduced pressure to yield the title compound. Yield: 35 mg.

The following compounds were prepared by the above procedure using the appropriate corresponding reagents.
2-[2-Methoxy-5-(1-oxa-2-azaspiro[4.4]non-2-en-3-yl)phenoxy]acetamide (Compounds No. 30)
Mass (m/z): 305.2 (M⁺+1).
2-[2-Methoxy-5-(5-oxa-6-azaspiro[3.4]oct-6-en-7-yl)phenoxy]acetamide (Compound No. 35) Mass (m/z): 291.3 (M⁺+1).
2-[2-Methoxy-5-(1-oxa-2-azaspiro[4.5]dec-2-en-3-yl)phenoxy]acetamide (Compound No. 37) Mass (m/z): 319.3 (M⁺+1).
2-[2-Methoxy-5-(1-oxa-2-azaspiro[4.4]non-2-en-3-yl)phenoxy]-*N,N*-dimethylacetamide (Compound No. 58)
Mass (m/z): 369.2 (M⁺+ Na).

### Step g: Synthesis of compound of Formula Xa

Methanolic hydrochloric acid solution (10 mL) was added to a solution of the compound *tert*-butyl{3-[2-methoxy-5-(1-oxa-2-azaspiro[4.5]dec-2-en-3-yl)phenoxy]propyl} carbamate (Compound No. 41) (50 mg, 0.0001196 mol) and stirred for 3 hours at room temperature. The reaction mixture was concentrated under reduced pressure. The residue thus obtained was washed with ether twice and dried to yield the title compound. Yield: 20 mg.

The following compound was prepared by the above procedure using the appropriate corresponding reagents.
Hydrochloride salt of 3-[2-Methoxy-5-(1-oxa-2-aza-spiro[4.5]dec-2-en-3-yl)-phenoxy]-propylamine (Compound No. 51)

### Scheme II

### Example 2: General procedure for the synthesis of Formula XIII

### Step a: Synthesis of compound of Formula XII

Sodium ethane thiolate (780 g, 0.00928 mol) was added to a solution of a compound of Formula XI (prepared following the procedure described in WO 05/021515) (0.00265 mol) in dimethylacetamide (10 mL) and the reaction mixture was stirred at 110 °C for 7-9 hours under nitrogen atmosphere. The mixture was quenched with aqueous ammonium chloride and extracted with ethyl acetate. The organic layer was washed with water, dried over anhydrous sodium sulphate and concentrated under reduced pressure to yield the title compound.

The following compounds were prepared by the above procedure using the appropriate corresponding reagents.
2-(2,3-Dihydro-1*H*-inden-2-yloxy)-4-(5-oxa-6-azaspiro[3.4]oct-6-en-7-yl)phenol (Compound No. 2)
Mass (m/z): 336.1 (M⁺+1).
2-(2,3-Dihydro-1*H*-inden-2-yloxy)-4-(1-oxa-2-azaspiro[4.4]non-2-en-3-yl)phenol (Compound No. 5)
Mass (m/z): 350.2 (M⁺+1).
2-(2,3-Dihydro-1*H*-inden-2-yloxy)-4-(1-oxa-2-azaspiro[4.5]dec-2-en-3-yl)phenol (Compound No. 8)
Mass (m/z): 364.2 (M⁺+1).

### Step b: Synthesis of compound of Formula XIII

Potassium carbonate (53 mg, 0.00038 mol) was added to a solution of a compound of Formula XII (0.000192 mol) in dimethylformamide (2 mL) and heated the reaction mixture to 60 °C. To the resulting mixture was added a compound of Formula C'-hal (0.00028 mol) dropwise and the reaction mixture was stirred at 70-80 °C for 10 hours. The reaction mixture was diluted with water and extracted with ethyl acetate. The organic extracts were collected, washed with brine, dried over anhydrous sodium sulphate and concentrated under reduced pressure. The residue thus obtained was purified by column chromatography to yield the title compound.

The following compounds were prepared by the above procedure using appropriate corresponding reagents.
7-[3-(2,3-Dihydro-1*H*-inden-2-yloxy)-4-methoxyphenyl]-5-oxa-6-azaspiro[3.4]oct-6-ene (Compound No. 1)
Mass (m/z): 350.2 (M⁺+1).
3-[3-(2,3-Dihydro-1*H*-inden-2-yloxy)-4-propoxyphenyl]-1-oxa-2-azaspiro[4.4]non-2-ene (Compound No. 3)
Mass (m/z): 392.2 (M⁺+1).
3-[3-(2,3-Dihydro-1*H*-inden-2-yloxy)-4-isopropoxyphenyl]-1-oxa-2-azaspiro[4.4]non-2-ene (Compound No. 4)
Mass (m/z): 392.2 (M⁺+1).
3-[3-(2,3-Dihydro-1*H*-inden-2-yloxy)-4-isobutoxyphenyl]-1-oxa-2-azaspiro[4.4]non-2-ene (Compound No. 6)
Mass (m/z): 406.2 (M⁺+1).
7-[3-(2,3-Dihydro-1*H*-inden-2-yloxy)-4-ethoxyphenyl]-5-oxa-6-azaspiro[3.4]oct-6-ene (Compound No. 7)
Mass (m/z): 364.2 (M⁺+1).
3-[4-Butoxy-3-(2,3-dihydro-1*H*-inden-2-yloxy)phenyl]-1-oxa-2-azaspiro[4.5]dec-2-ene (Compound No. 9)
Mass (m/z): 420.2 (M⁺+1).
3-[3-(2,3-Dihydro-1*H*-inden-2-yloxy)-4-propoxyphenyl]-1-oxa-2-azaspiro[4.5]dec-2-ene (Compound No. 10)
Mass (m/z): 406.2 (M⁺+1).
3-[4-(Difluoromethoxy)-3-(2,3-dihydro-1*H*-inden-2-yloxy)phenyl]-1-oxa-2-azaspiro[4.5]dec-2-ene (Compound No. 11)
Mass (m/z): 414.1 (M⁺+1).
3-[3-(2,3-Dihydro-1*H*-inden-2-yloxy)-4-ethoxyphenyl]-1-oxa-2-azaspiro[4.5]dec-2-ene (Compound No. 12)
Mass (m/z): 392.2 (M⁺+1).
3-[4-(Cyclopropylmethoxy)-3-(2,3-dihydro-1*H*-inden-2-yloxy)phenyl]-1-oxa-2-azaspiro[4.5]dec-2-ene (Compound No. 13)
Mass (m/z): 418.2 (M⁺+1).
3-[3-(2,3-Dihydro-1*H*-inden-2-yloxy)-4-isopropoxyphenyl]-1-oxa-2-azaspiro[4.5]dec-2-ene (Compound No. 14)
Mass (m/z): 406.2 (M⁺+1).
3-[3-(2,3-Dihydro-1*H*-inden-2-yloxy)-4-methoxyphenyl]-1-oxa-2-azaspiro[4.5]dec-2-ene (Compound No. 15)
Mass (m/z): 378 (M⁺+1).
7-[3-(2,3-Dihydro-1*H*-inden-2-yloxy)-4-isopropoxyphenyl]-5-oxa-6-azaspiro[3.4]oct-6-ene (Compound No. 16)
Mass (m/z): 378.1 (M⁺+1).
3-[4-Butoxy-3-(2,3-dihydro-1*H*-inden-2-yloxy)phenyl]-1-oxa-2-azaspiro[4.4]non-2-ene (Compound No. 17)
Mass (m/z): 406.1 (M⁺+1).
3-[4-(Cyclopropylmethoxy)-3-(2,3-dihydro-1*H*-inden-2-yloxy)phenyl]-1-oxa-2-azaspiro[4.4]non-2-ene (Compound No. 18)
Mass (m/z): 404.1 (M⁺+1).
7-[3-(2,3-Dihydro-1*H*-inden-2-yloxy)-4-propoxyphenyl]-5-oxa-6-azaspiro[3.4]oct-6-ene (Compound No. 19)
Mass (m/z): 378.1 (M⁺+1).
7-[4-Butoxy-3-(2,3-dihydro-1*H*-inden-2-yloxy)phenyl]-5-oxa-6-azaspiro[3.4]oct-6-ene (Compound No. 20)
Mass (m/z): 392.2 (M⁺+1).
3-[2-(2,3-Dihydro-1*H*-inden-2-yloxy)-4-(1-oxa-2-azaspiro[4.4]non-2-en-3-yl)phenoxy]propan-1-ol (Compound No. 21)
Mass (m/z): 394.1 (M⁺+1).
3-[3-(2,3-Dihydro-1*H*-inden-2-yloxy)-4-(2-morpholin-4-ylethoxy)phenyl]-1-oxa-2-azaspiro[4.4]non-2-ene (Compound No. 22)
Mass (m/z): 463.2 (M⁺+1).
[2-(2,3-Dihydro-1*H*-inden-2-yloxy)-4-(1,7-dioxa-2-azaspiro[4.4]non-2-en-3-yl)phenoxy] ethanol (Compound No. 23)
Mass (m/z): 396.2 (M⁺+1).
3-[3-(2,3-Dihydro-1*H*-inden-2-yloxy)-4-methoxyphenyl]-1-oxa-2-azaspiro[4.4]non-2-ene (Compound No. 24)
Mass (m/z): 364.1 (M⁺+1).
7-[4-(Difluoromethoxy)-3-(2,3-dihydro-1*H*-inden-2-yloxy)phenyl]-5-oxa-6-azaspiro[3.4]oct-6-ene (Compound No. 25)
Mass (m/z): 386.1 (M⁺+1).

### Scheme III:

### Example 3a: General procedure for the synthesis of compound of Formula XVI (wherein G in compound of Formula XV is hal)

Potassium carbonate (74 mg, 0.0005 mol) was added to a solution of a compound of Formula XIV (0.00026 mol) in dimethylformamide (2 mL) and heated the reaction mixture to 60 °C. A compound of Formula XV (0.00026 mol) was added to the resulting mixture and the reaction mixture was stirred at 50-60 °C for 8 hours. The reaction mixture was diluted with water and extracted with ethyl acetate. The organic extracts were collected, washed with brine, dried over anhydrous sodium sulphate and concentrated under reduced pressure. The residue thus obtained was purified by column chromatography to yield the title compound.

The following compounds were prepared by the above procedure using the appropriate corresponding reagents.
3-[2-Methoxy-5-(1-oxa-2-azaspiro[4.5]dec-2-en-3-yl)phenoxy]propan-1-ol (Compound No. 47)
Mass (m/z): 306.2 (M⁺+1).
3-[5-(1,7-Dioxa-2-azaspiro[4.4]non-2-en-3-yl)-2-methoxyphenoxy]propan-1-ol (Compound No. 48)
Mass (m/z): 294.2 (M⁺+1).
5-(1,8-Dioxa-2-azaspiro[4.5]dec-2-en-3-yl)-2-methoxyphenyl methanesulfonate (Compound No. 49)
Mass (m/z): 327.95(M⁺+1).
2-Methoxy-5-(1-oxa-2-azaspiro[4.5]dec-2-en-3-yl)phenyl methanesulfonate (Compound No. 50)
Mass (m/z): 339.98 (M⁺+1).

### Example 3b: General procedure for the synthesis of compound of Formula XVI (wherein G in compound of Formula XV is -OH)

Diisopropylazodicarboxylate (0.41 mL, 0.00207) was added to a solution of a compound of Formula XIV (0.00173 mol), triphenylphosphine (498 mg, 0.00189 mol) and a compound of Formula XV (0.00189 mol) in tetrahydrofuran. The reaction mixture was stirred at room temperature overnight. The organic solvent was removed under reduced pressure and the residue thus obtained was purified by column chromatography to yield the title compound.

The following compounds were prepared by the above procedure using the appropriate corresponding reagents.
3-[3-({4-[(Benzyloxy)methyl] cyclohexyl}methoxy)-4-methoxyphenyl]-1,7-dioxa-2-azaspiro[4.4]non-2-ene (Compound No. 45)
Mass (m/z): 466.2 (M⁺+1).
*Tert-*butyl4-{[2-methoxy-5-(1-oxa-2-azaspiro[4.4]non-2-en-3-yl)phenoxy]methyl}piperidine-1-carboxylate (Compound No. 46)
Mass (m/z): 467.2 (M⁺+ 23).

### Scheme IV

### Example 4: (S)-3-[3-(Indan-2-yloxy)-4-methoxy-phenyl]-1 ,7-dioxa-2-aza-spiro[4.4]non-2-ene (Compound No. 52)

### Step a: Synthesis of L-Ephederine salt of 5-(carboxymethyl)-3-[3-(2,3-dihydro-1H-inden-2-yloxy)-4-methoxyphenyl]-4,5-dihydroisoxazole-5-carboxylic acid

5-(Carboxymethyl)-3-[3-(cyclopentyloxy)-4-methoxyphenyl]-4,5-dihydroisoxazole-5-carboxylic acid (prepared following the procedure as described in WO 05/021515) (7 g, 0.017 mol) and L-Ephederine (5.63 g, 0.034 mol) were dissolved in acetone (300 mL) and the mixture was refluxed for 4 hours. The reaction mixture was slowly brought to room temperature and maintained at room temperature for 24-36 hours to yield the title compound. Yield: 9.3 g.

### Step b: Synthesis of (S)-methyl 3-[3-(2,3-dihydro-1H-inden-2-yloxy)-4-methoxyphenyl]-5-(2-methoxy-2-oxoethyl)-4,5-dihydroisoxazole-5-carboxylate

Thionyl chloride (27.3 mL, 0.377 mol) was added slowly to a dry methanol solution (300 mL) at 0 °C under nitrogen atmosphere and stirred for 1 hour. The compound obtained from step *a* above (9.3 g, 0.0126 mol) was added to the resulting reaction mixture at 0°C. The reaction mixture was slowly brought to room temperature and stirred at room temperature for 12 hours. The reaction mixture was concentrated followed by extraction with ethyl acetate. The organic portion was washed with water, brine and dried over anhydrous sodium sulphate and concentrated under reduced pressure. The residue thus obtained was purified by column chromatography to yield the title compound. Yield: 5.35 g.

### Step c: Synthesis of (S)-2-[3-[3-(2,3-dihydro-1H-inden-2-yloxy)-4-methoxyphenyl]-5-(hydroxymethyl)-4,5-dihydroisoxazol-5-yl]ethanol

The compound obtained from step *b* above (3.7 g, 0.008 mol) was dissolved in tetrahydrofuran (150 mL) and cooled to 0 °C and lithium aluminum hydride (0.96 g, 0.025 mol) was added portion wise. The reaction mixture was stirred for 1 hour. Reaction mixture was quenched with aqueous solution of sodium sulphate solution (20 %, 10 mL) at 0 °C and filtered through celite. Extraction was done with ethyl acetate to obtain the crude product. The compound was purified by column chromatography to yield the title compound. Yield: 2.8 g.

### Step d: (S)-3-[3-(Indan-2-yloxy)-4-methoxy-phenyl]-1,7-dioxa-2-aza-spiro[4.4]non-2-ene (Compound No. 52)

Dry tetrahydrofuran (80 mL) was added to a solution of the compound obtained from step c above (3 g, 0.0078 mol), triphenyl phosphine (2.65 g, 0.010 mol) and succinimide (0.93 g, 0.0093 mol) and the reaction mixture was stirred for 20 minutes at room temperature and then subsequently cooled to 0 °C. Diisopropylazodicarboxylate (2.43 mL, 0.0124 mol) was added slowly over a period of 10 minutes at 0 °C and further stirred at room temperature overnight. The reaction mixture was concentrated under reduced pressure. The residue thus obtained was purified by column chromatography to yield the title compound. Yield: 0.28 g. m.p.: 137.6-138 °C;
[α]_{D} = -0.681 (c, 1.03, CH₃CN);
¹H NMR (CDCl₃) δ 7.44 (s, 1 H), 7.26 (m, 4 H), 7.01 (d, *J* = 0.02 Hz, 1 H), 6.85 (d, *J* = 0.02 Hz, 1 H), 4.82 (m, 1 H), 4.11 (m, 1 H), 4.04 (m, 2 H), 3.85 (s, 3 H), 3.83 (m, 1 H), 3.4 (m, 4 H), 3.2(d, 2 H), 2.46 (m, 1 H), 2.08 (m, 1 H);
Mass: 366 (M+1);
Chiral HPLC = 99 % (CHIRALCEL OD).

Following compound can be prepared by the above procedure by using D-Ephederine in place of L-Ephederine.

### (R)-3-[3-(Indan-2-yloxy)-4-methoxy-phenyl]-1,7-dioxa-2-aza-spiro[4.4]non-2-ene (Compound No. 53)

m.p.: 137.6-138 °C;
[α]_{D} = + 0.873(c, 1, CH₃CN);
¹H NMR (CDCl₃) δ 7.44 (s, 1 H), 7.26 (m, 4 H), 7.01 (d, *J* = 0.02 Hz, 1 H), 6.85 (d, *J* = 0.02 Hz, 1 H), 4.82 (m, 1 H), 4.11 (m, 1 H), 4.04 (m, 2 H), 3.85 (s, 3 H), 3.83 (m, 1 H), 3.4 (m, 4 H), 3.2(d, 2 H), 2.46 (m, 1 H), 2.08 (m, 1 H);
Mass: 366 (M+1);
HPLC = 99 % (CHIRALCEL OD)

### PDE-IV Enzyme Assay

The efficacy of compounds as PDE-4 inhibitor was determined by an enzyme assay (Burnouf et al.; J. Med. Chem., 2000, 43:4850-4867). The PDE-4 enzyme source used was U937 cell cytosolic fraction prepared by sonication. The enzyme reaction was carried out, with the cytosolic fraction as the enzyme source, in the presence of cAMP (1 µM) at 30 °C in the presence or absence of NCE for 45 -60 min. An aliquot of this reaction mixture was taken further for the ELISA assay to determine level of cAMP in the sample. The concentration of the cAMP in the sample directly correlates with the degree of PDE-4 enzyme inhibition. Results were expressed as percent control. IC₅₀ values of test compounds ranged from between about 1.5 nM to greater than about 10 µM, from between about 1.5 nM to about 1100 nM, from between about 1.5 nM to about 500 nM, and even from between about 4 nM to about 350 nM.

## Claims

1. A compound having the structure of Formula I, 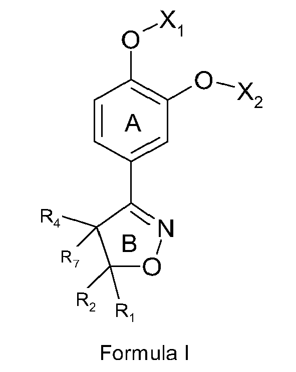 or its pharmaceutically acceptable salts, pharmaceutically acceptable solvates, enantiomers, diastereomers or N-oxides, wherein
R₁ and R₂ together form an optionally substituted cycloalkyl or heterocyclyl ring, wherein the optional substituent is oxo, alkyl, alkenyl, alkynyl, halogen, nitro, -NH₂, -C(=O)NRₓR_{y}, -NHCOOR₆, cyano, hydroxy, alkoxy, or substituted amino;
R₄ is hydrogen, alkyl, hydroxy, halogen or carboxy;
R₇ is hydrogen or alkyl;
X₁ and X₂ is hydrogen, alkyl, cycloalkyl, alkaryl, cycloalkylalkyl, heteroaryl, heterocyclyl, heteroarylalkyl, heterocyclylalkyl, -SO₂R₅, -(CH₂)_{g}NHCOOalkyl, -(CH₂)_{g}C(=O)NRₓR_{y} or - (CH₂)_{g1}C(=O)OR₃ (wherein g is an integer from 0-3 and g₁ is an integer from 1-3);
X₁ and X₂ together optionally form a cyclic ring fused with ring A of Formula I, wherein ring
A contains 3-5 carbon atoms and 2-3 heteroatoms selected from N, O or S;
wherein
R₃ is alkyl, cycloalkyl or heterocyclyl;
halogen is F, Cl, Br, or I;
Rₓ and R_{y} each independently is hydrogen, alkyl, C₃-C₆ alkenyl, C₃-C₆ alkynyl, carboxy, cycloalkyl, -S(O)ₘR₅, aryl, alkaryl, heteroaryl, heterocyclyl, heteroarylalkyl or heterocyclylalkyl;
m is an integer between 0-2;
R₆ is alkyl, alkenyl, alkynyl, cycloalkyl, alkaryl, heteroarylalkyl or heterocyclylalkyl; and
R₅ is hydrogen, alkyl, alkenyl, alkynyl, aryl, cycloalkyl, alkaryl, heteroaryl, heteroarylalkyl, heterocyclyl or heterocyclylalkyl.

2. A compound selected from:
7-[3-(2,3-dihydro-1*H*-inden-2-yloxy)-4-methoxyphenyl]-5-oxa-6-azaspiro[3.4]oct-6-ene (Compound No. 1);
2-(2,3-dihydro-1*H*-inden-2-yloxy)-4-(5-oxa-6-azaspiro[3.4]oct-6-en-7-yl)phenol (Compound No. 2);
3-[3-(2,3-dihydro-1*H*-inden-2-yloxy)-4-propoxyphenyl]-1-oxa-2-azaspiro[4.4]non-2-ene (Compound No. 3);
3-[3-(2,3-dihydro-1*H*-inden-2-yloxy)-4-isopropoxyphenyl]-1-oxa-2-azaspiro[4.4]non-2-ene (Compound No. 4);
2-(2,3-dihydro-1*H*-inden-2-yloxy)-4-(1-oxa-2-azaspiro[4.4]non-2-en-3-yl)phenol (Compound No. 5);
3-[3-(2,3-Dihydro-1*H*-inden-2-yloxy)-4-isobutoxyphenyl]-1-oxa-2-azaspiro[4.4]non-2-ene (Compound No. 6);
7-[3-(2,3-Dihydro-1*H*-inden-2-yloxy)-4-ethoxyphenyl]-5-oxa-6-azaspiro[3.4]oct-6-ene (Compound No. 7);
2-(2,3-Dihydro-1*H*-inden-2-yloxy)-4-(1-oxa-2-azaspiro[4.5]dec-2-en-3-yl)phenol (Compound No. 8)
3-[4-Butoxy-3-(2,3-dihydro-1*H*-inden-2-yloxy)phenyl]-1-oxa-2-azaspiro[4.5]dec-2-ene (Compound No. 9);
3-[3-(2,3-Dihydro-1*H*-inden-2-yloxy)-4-propoxyphenyl]-1-oxa-2-azaspiro[4.5]dec-2-ene (Compound No. 10);
3-[4-(Difluoromethoxy)-3-(2,3-dihydro-1*H*-inden-2-yloxy)phenyl]-1-oxa-2-azaspiro[4.5]dec-2-ene (Compound No. 11);
3-[3-(2,3-Dihydro-1*H*-inden-2-yloxy)-4-ethoxyphenyl]-1-oxa-2-azaspiro[4.5]dec-2-ene (Compound No. 12);
3-[4-(Cyclopropylmethoxy)-3-(2,3-dihydro-1*H* inden-2-yloxy)phenyl]-1-oxa-2-azaspiro[4.5]dec-2-ene (Compound No. 13);
3-[3-(2,3-Dihydro-1*H*-inden-2-yloxy)-4-isopropoxyphenyl]-1-oxa-2-azaspiro[4.5]dec-2-ene (Compound No. 14);
3-[3-(2,3-Dihydro-1*H*-inden-2-yloxy)-4-methoxyphenyl]-1-oxa-2-azaspiro[4.5]dec-2-ene (Compound No. 15);
7-[3-(2,3-Dihydro-1*H*-inden-2-yloxy)-4-isopropoxyphenyl]-5-oxa-6-azaspiro[3.4]oct-6-ene (Compound No. 16);
3-[4-Butoxy-3-(2,3-dihydro-1*H*-inden-2-yloxy)phenyl]-1-oxa-2-azaspiro[4.4]non-2-ene (Compound No. 17);
3-[4-(Cyclopropylmethoxy)-3-(2,3-dihydro-1*H*-inden-2-yloxy)phenyl]-1-oxa-2-azaspiro[4.4]non-2-ene (Compound No. 18);
7-[3-(2,3-Dihydro-1*H*-inden-2-yloxy)-4-propoxyphenyl]-5-oxa-6-azaspiro[3.4]oct-6-ene (Compound No. 19);
7-[4-butoxy-3-(2,3-dihydro-1*H*-inden-2-yloxy)phenyl]-5-oxa-6-azaspiro[3.4]oct-6-ene (Compound No. 20);
3-[2-(2,3-Dihydro-1*H*-inden-2-yloxy)-4-(1-oxa-2-azaspiro[4.4]non-2-en-3-yl)phenoxy]propan-1-ol (Compound No. 21);
3-[3-(2,3-Dihydro-1*H*-inden-2-yloxy)-4-(2-morpholin-4-ylethoxy)phenyl]-1-oxa-2-azaspiro[4.4]non-2-ene (Compound No. 22);
[2-(2,3-Dihydro-1*H*-inden-2-yloxy)-4-(1,7-dioxa-2-azaspiro[4.4]non-2-en-3-yl)phenoxy]ethanol (Compound No. 23)
3-[3-(2,3-Dihydro-1*H*-inden-2-yloxy)-4-methoxyphenyl]-1-oxa-2-azaspiro[4.4]non-2-ene (Compound No. 24);
7-[4-(Difluoromethoxy)-3-(2,3-dihydro-1*H*-inden-2-yloxy)phenyl]-5-oxa-6-azaspiro[3.4]oct-6-ene (Compound No. 25);
Ethyl [2-methoxy-5-(1-oxa-2-azaspiro[4.5]dec-2-en-3-yl)phenoxy]acetate (Compound No. 26);
2-[2-Methoxy-5-(1-oxa-2-azaspiro[4.5]dec-2-en-3-yl)phenoxy]-*N*-methylacetamide (Compound No. 27);
Ethyl [2-methoxy-5-(1-oxa-2-azaspiro[4.4]non-2-en-3-yl)phenoxy]acetate (Compound No. 28);
[2-Methoxy-5-(1-oxa-2-azaspiro[4.4]non-2-en-3-yl)phenoxy]acetic acid (Compound No. 29);
2-[2-Methoxy-5-(1-oxa-2-azaspiro[4.4]non-2-en-3-yl)phenoxy]acetamide (Compound No. 30);
2-[2-Methoxy-5-(1-oxa-2-azaspiro[4.4]non-2-en-3-yl)phenoxy]-*N*-methylacetamide (Compound No. 31);
*N*-Cyclopentyl-2-[2-methoxy-5-(1-oxa-2-azaspiro[4.4]non-2-en-3-yl)phenoxy]acetamide (Compound No. 32);
Ethyl [2-methoxy-5-(5-oxa-6-azaspiro[3.4]oct-6-en-7-yl)phenoxy]acetate (Compound No. 33);
[2-Methoxy-5-(5-oxa-6-azaspiro[3.4]oct-6-en-7-yl)phenoxy]acetic acid (Compound No. 34);
2-[2-Methoxy-5-(5-oxa-6-azaspiro[3.4]oct-6-en-7-yl)phenoxy]acetamide (Compound No. 35);
2-[2-Methoxy-5-(5-oxa-6-azaspiro[3.4]oct-6-en-7-yl)phenoxy]-*N*-methylacetamide (Compound No. 36);
2-[2-Methoxy-5-(1-oxa-2-azaspiro[4.5]dec-2-en-3-yl)phenoxy]acetamide (Compound No. 37);
[5-(1,7-Dioxa-2-azaspiro[4.4]non-2-en-3-yl)-2-methoxyphenoxy]acetic acid (Compound No. 38);
*Tert*-butyl {3-[2-methoxy-5-(5-oxa-6-azaspiro[3.4]oct-6-en-7-yl)phenoxy]propyl} carbamate (Compound No. 39);
*Tert-*butyl {3 -[2-methoxy-5-(1-oxa-2-azaspiro[4.4]non-2-en-3-yl)phenoxy]propyl} carbamate (Compound No. 40);
*Tert*-butyl {3 -[2-methoxy-5-(1-oxa-2-azaspiro[4.5]dec-2-en-3 -yl)phenoxy]propyl} carbamate (Compound No. 41);
Methyl 5-[2-methoxy-5-(5-oxa-6-azaspiro[3.4]oct-6-en-7-yl)phenoxy]pentanoate (Compound No. 42);
Methyl 5-[2-methoxy-5-(1-oxa-2-azaspiro[4.5]dec-2-en-3-yl)phenoxy]pentanoate (Compound No. 43);
Methyl 5-[2-methoxy-5-(1-oxa-2-azaspiro[4.4]non-2-en-3-yl)phenoxy]pentanoate (Compound No. 44);
3-[3-({4-[(Benzyloxy)methyl]cyclohexyl}methoxy)-4-methoxyphenyl]-1,7-dioxa-2-azaspiro[4.4]non-2-ene (Compound No. 45)
*Tert*-butyl 4-{[2-methoxy-5-(1-oxa-2-azaspiro[4.4]non-2-en-3-yl)phenoxy]methyl}piperidine-1-carboxylate (Compound No. 46)
3-[2-Methoxy-5-(1-oxa-2-azaspiro[4.5]dec-2-en-3-yl)phenoxy]propan-1-ol (Compound No. 47);
3-[5-(1,7-Dioxa-2-azaspiro[4.4]non-2-en-3-yl)-2-methoxyphenoxy]propan-1-ol (Compound No. 48);
5-(1,8-Dioxa-2-azaspiro[4.5]dec-2-en-3-yl)-2-methoxyphenyl methanesulfonate (Compound No. 49);
2-Methoxy-5-(1-oxa-2-azaspiro[4.5]dec-2-en-3-yl)phenyl methanesulfonate (Compound No. 50);
Hydrochloride salt of 3-[2-Methoxy-5-(1-oxa-2-aza-spiro[4.5]dec-2-en-3-yl)-phenoxy]-propylamine (Compound No. 51)
(S)-3-[3-(Indan-2-yloxy)-4-methoxy-phenyl]-1,7-dioxa-2-aza-spiro[4.4]non-2-ene (Compound No. 52);
(R)-3-[3-(Indan-2-yloxy)-4-methoxy-phenyl]-1,7-dioxa-2-aza-spiro[4.4]non-2-ene (Compound No. 53);
3-[3-(2,3-Dihydro-1*H* inden-1-yloxy)-4-methoxyphenyl]-1-oxa-2-azaspiro[4.5]dec-2-ene (Compound No. 54);
3-[3-(2,3-Dihydro-1*H*-inden-1-yloxy)-4-methoxyphenyl]-1-oxa-2-azaspiro[4.4]non-2-ene (Compound No. 55);
7-[3-(2,3-Dihydro-1*H*-inden-1-yloxy)-4-methoxyphenyl]-5-oxa-6-azaspiro[3.4]oct-6-ene (Compound No. 56);
3-[3-(Benzyloxy)-4-methoxyphenyl]-1-oxa-2-azaspiro[4.5]dec-2-ene (Compound No. 57);
2-[2-Methoxy-5-(1-oxa-2-azaspiro[4.4]non-2-en-3-yl)phenoxy]-*N,N*-dimethylacetamide (Compound No. 58).

3. A pharmaceutical composition comprising a therapeutically effective amount of a compound having the structure of Formula I, 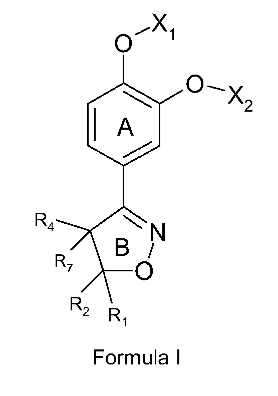 or its pharmaceutically acceptable salts, pharmaceutically acceptable solvates, enantiomers, diastereomers or N-oxides, together with one or more pharmaceutically acceptable excipient, carrier or diluent, wherein
R₁ and R₂ together form an optionally substituted cycloalkyl or heterocyclyl ring, wherein the optional substituent is oxo, alkyl, alkenyl, alkynyl, halogen, nitro, -NH₂, -C(=O)NRₓR_{y}, -NHCOOR₆, cyano, hydroxy, alkoxy, or substituted amino;
R₄ is hydrogen, alkyl, hydroxy, halogen or carboxy;
R₇ is hydrogen or alkyl;
X₁ and X₂ is hydrogen, alkyl, cycloalkyl, alkaryl, cycloalkylalkyl, heteroaryl, heterocyclyl, heteroarylalkyl, heterocyclylalkyl, -SO₂R₅, -(CH₂)_{g}NHCOOalkyl, -(CH₂)_{g}C(=O)NRₓR_{y} or -(CH₂)_{g1}C(=O)OR₃ (wherein g is an integer from 0-3 and g₁ is an integer from 1-3);
X₁ and X₂ together optionally form a cyclic ring fused with ring A of Formula I, wherein ring
A contains 3-5 carbon atoms and 2-3 heteroatoms selected from N, O or S;
wherein
R₃ is alkyl, cycloalkyl or heterocyclyl;
halogen is F, Cl, Br, or I;
Rₓ and R_{y} each independently is hydrogen, alkyl, C₃-C₆ alkenyl, C₃-C₆ alkynyl, carboxy, cycloalkyl, -S(O)ₘR₅, aryl, alkaryl, heteroaryl, heterocyclyl, heteroarylalkyl or heterocyclylalkyl;
m is an integer between 0-2;
R₆ is alkyl, alkenyl, alkynyl, cycloalkyl, alkaryl, heteroarylalkyl or heterocyclylalkyl; and
R₅ is hydrogen, alkyl, alkenyl, alkynyl, aryl, cycloalkyl, alkaryl, heteroaryl, heteroarylalkyl, heterocyclyl or heterocyclylalkyl.

4. A method of treating AIDS, asthma, arthritis, bronchitis, chronic obstructive pulmonary disease (COPD), psoriasis, allergic rhinitis, shock, atopic dermatitis, Crohn's disease, adult respiratory distress syndrome (ARDS), eosinophilic granuloma, allergic conjunctivitis, osteoarthritis or ulcerative colitis comprising administering to an animal or human in need thereof a therapeutically effective amount of a compound having the structure of Formula I, 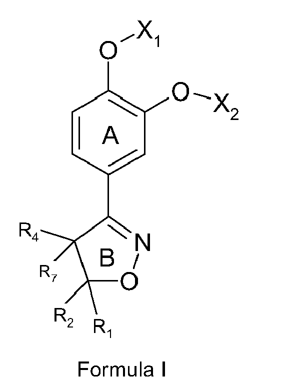 or its pharmaceutically acceptable salts, pharmaceutically acceptable solvates, enantiomers, diastereomers or N-oxides, wherein
R₁ and R₂ together form an optionally substituted cycloalkyl or heterocyclyl ring, wherein the optional substituent is oxo, alkyl, alkenyl, alkynyl, halogen, nitro, -NH₂, -C(=O)NRₓR_{y}, -NHCOOR₆, cyano, hydroxy, alkoxy, or substituted amino;
R₄ is hydrogen, alkyl, hydroxy, halogen or carboxy;
R₇ is hydrogen or alkyl;
X₁ and X₂ is hydrogen, alkyl, cycloalkyl, alkaryl, cycloalkylalkyl, heteroaryl, heterocyclyl, heteroarylalkyl, heterocyclylalkyl, -SO₂R₅, -(CH₂)_{g}NHCOOalkyl, -(CH₂)_{g}C(=O)NRₓR_{y} or -(CH₂)_{g1}C(=O)OR₃ (wherein g is an integer from 0-3 and g₁ is an integer from 1-3);
X₁ and X₂ together optionally form a cyclic ring fused with ring A of Formula I, wherein ring
A contains 3-5 carbon atoms and 2-3 heteroatoms selected from N, O or S;
wherein
R₃ is alkyl, cycloalkyl or heterocyclyl;
halogen is F, Cl, Br, or I;
Rₓ and R_{y} each independently is hydrogen, alkyl, C₃-C₆ alkenyl, C₃-C₆ alkynyl, carboxy, cycloalkyl, -S(O)ₘR₅, aryl, alkaryl, heteroaryl, heterocyclyl, heteroarylalkyl or heterocyclylalkyl;
m is an integer between 0-2;
R₆ is alkyl, alkenyl, alkynyl, cycloalkyl, alkaryl, heteroarylalkyl or heterocyclylalkyl; and
R₅ is hydrogen, alkyl, alkenyl, alkynyl, aryl, cycloalkyl, alkaryl, heteroaryl, heteroarylalkyl, heterocyclyl or heterocyclylalkyl.

5. A method of preventing, inhibiting or suppressing an inflammatory condition comprising administering to an animal or human in need thereof a therapeutically effective amount of a compound having the structure of Formula I, 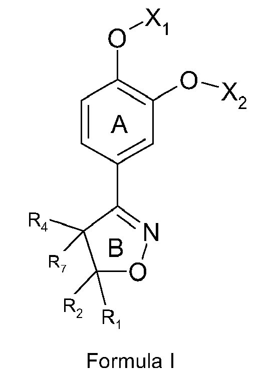 or its pharmaceutically acceptable salts, pharmaceutically acceptable solvates, enantiomers, diastereomers or N-oxides, wherein
R₁ and R₂ together form an optionally substituted cycloalkyl or heterocyclyl ring, wherein the optional substituent is oxo, alkyl, alkenyl, alkynyl, halogen, nitro, -NH₂, -C(=O)NRₓR_{y}, -NHCOOR₆, cyano, hydroxy, alkoxy, or substituted amino;
R₄ is hydrogen, alkyl, hydroxy, halogen or carboxy;
R₇ is hydrogen or alkyl;
X₁ and X₂ is hydrogen, alkyl, cycloalkyl, alkaryl, cycloalkylalkyl, heteroaryl, heterocyclyl, heteroarylalkyl, heterocyclylalkyl, -SO₂R₅, -(CH₂)_{g}NHCOOalkyl, -(CH₂)_{g}C(=O)NRₓR_{y} or -(CH₂)_{g1}C(=O)OR₃ (wherein g is an integer from 0-3 and g₁ is an integer from 1-3);
X₁ and X₂ together optionally form a cyclic ring fused with ring A of Formula I, wherein ring
A contains 3-5 carbon atoms and 2-3 heteroatoms selected from N, O or S;
wherein
R₃ is alkyl, cycloalkyl or heterocyclyl;
halogen is F, Cl, Br, or I;
Rₓ and R_{y} each independently is hydrogen, alkyl, C₃-C₆ alkenyl, C₃-C₆ alkynyl, carboxy, cycloalkyl, -S(O)ₘR₅, aryl, alkaryl, heteroaryl, heterocyclyl, heteroarylalkyl or heterocyclylalkyl;
m is an integer between 0-2;
R₆ is alkyl, alkenyl, alkynyl, cycloalkyl, alkaryl, heteroarylalkyl or heterocyclylalkyl; and
R₅ is hydrogen, alkyl, alkenyl, alkynyl, aryl, cycloalkyl, alkaryl, heteroaryl, heteroarylalkyl, heterocyclyl or heterocyclylalkyl.

6. A method for preparing a compound of Formulae VII, VIII, IX, X or Xa, or its pharmaceutically acceptable salts, pharmaceutically acceptable solvates, enantiomers, diastereomers or N-oxides comprising the steps of:
a. reacting a compound of Formula II 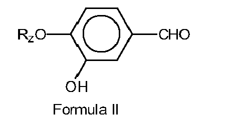 with a compound of Formula III 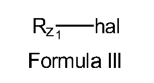 to form a compound of Formula IV, 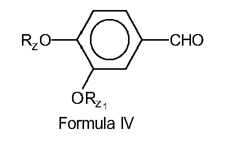
b. reacting the compound of Formula IV with hydroxylamine hydrochloride to form a compound of Formula V, and 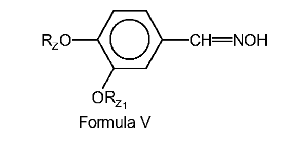
c. reacting the compound of Formula V with a compound of Formula VI 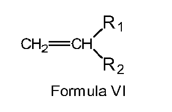 to form a compound of Formula VII, 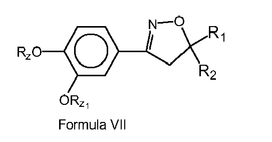
d. optionally hydrolyzing the compound of Formula VII (when Rz1 is -CH₂COOalkyl) to form a compound of Formula VIII, 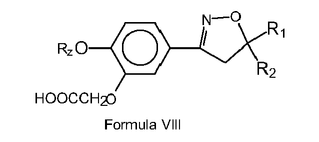
e. optionally reacting the compound of Formula VIII with a compound of Formula -NHRₓR_{y} to form a compound of Formula IX, 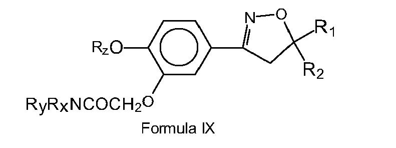
f. optionally reacting the compound of Formula VII with methanolic ammonia to form a compound of Formula X, 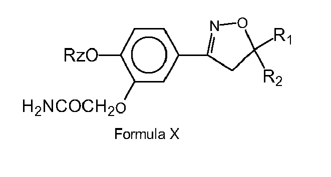
g. optionally deprotecting the compound of Formula VII (when -(CH₂)g1NHCOOalkyl) to form a compound of Formula Xa 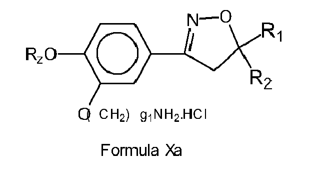
wherein
hal is Cl, Br or I;
R_{z} is alkyl optionally substituted with halogen or alkaryl;
R_{z1} is alkyl, cycloalkyl, alkaryl, alkenyl, cycloalkylalkyl, heteroaryl, heterocyclyl, heteroarylalkyl, heterocyclylalkyl or -(CH₂)_{g1}C(=O)OR₃;
R₁ and R₂ together form an optionally substituted cycloalkyl or heterocyclyl ring wherein the optional substituent is oxo, alkyl, alkenyl, alkynyl, halogen, nitro, -NH₂, -C(=O)NRₓR_{y}, -NHCOOR₆, cyano, hydroxy, alkoxy, or substituted amino; R₃ is alkyl, cycloalkyl or heterocyclyl;
g₁ is an integer from 1-3;
Rₓ and R_{y} each independently is hydrogen, alkyl, C₃-C₆ alkenyl, C₃-C₆ alkynyl, carboxy, cycloalkyl, -S(O)ₘR₅, aryl, alkaryl, heteroaryl, heterocyclyl, heteroarylalkyl or heterocyclylalkyl;
m is an integer between 0-2;
R₆ is alkyl, alkenyl, alkynyl, cycloalkyl, alkaryl, heteroarylalkyl or heterocyclylalkyl; and
R₅ is hydrogen, alkyl, alkenyl, alkynyl, aryl, cycloalkyl, alkaryl, heteroaryl, heteroarylalkyl, heterocyclyl or heterocyclylalkyl.

7. A method for preparing a compound of Formulae XII or XIII, or its pharmaceutically acceptable salts, pharmaceutically acceptable solvates, enantiomers, diastereomers or N-oxides, comprising the steps of:
a. demethylating a compound of Formula XI 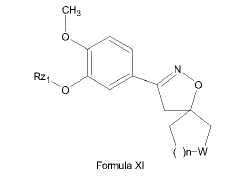 to form a compound of Formula XII, and 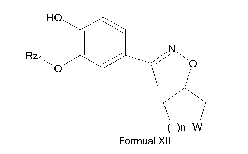
b. optionally reacting the compound of Formula XII with a compound of Formula C'-hal to form a compound of Formula XIII, 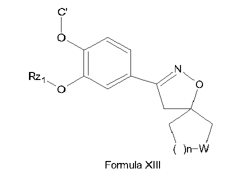
wherein
C' is heterocyclylalkyl, cycloalkylalkyl, hydroxyalkyl, cycloalkyl or C₂₋₁₀ alkyl optionally substituted with halogen;
hal is Cl, Br or I;
R_{z1} is alkyl, cycloalkyl, alkaryl, alkenyl, cycloalkylalkyl, heteroaryl, heterocyclyl, heteroarylalkyl, heterocyclylalkyl or -(CH₂)_{g1}C(=O)OR₃;
R₃ is alkyl, cycloalkyl or heterocyclyl;
g₁ is an integer from 1-3;
n is 0-3; and
W is oxygen or carbon

8. A method for preparing a compound of Formula XVI, or its pharmaceutically acceptable salts, pharmaceutically acceptable solvates, enantiomers, diastereomers or N-oxides, comprising the steps of:
a. reacting a compound of Formula XIV 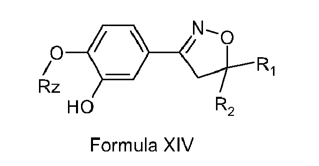 with a compound of Formula XV
Rw-G Formula XV
to form a compound of Formula XVI, 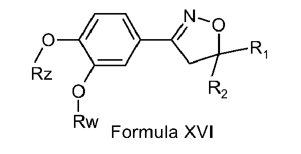
wherein
R_{w} is alkyl, cycloalkyl, heteroaryl, heterocyclyl or -SO₂R₅;
G is hal (wherein hal is Cl, Br or I) or -OH;
R_{z} is alkyl optionally substituted with halogen or alkaryl;
R₁ and R₂ together form an optionally substituted cycloalkyl or heterocyclyl ring, wherein the optional substituent is oxo, alkyl, alkenyl, alkynyl, halogen, nitro, -NH₂, -C(=O)NRₓR_{y}, -NHCOOR₆, cyano, hydroxy, alkoxy, or substituted amino;
R₃ is alkyl, cycloalkyl or heterocyclyl;
g₁ is an integer from 1-3;
Rₓ and R_{y} each independently is hydrogen, alkyl, C₃-C₆ alkenyl, C₃-C₆ alkynyl, carboxy, cycloalkyl, -S(O)ₘR₅, aryl, alkaryl, heteroaryl, heterocyclyl, heteroarylalkyl or heterocyclylalkyl;
m is an integer between 0-2;
R₆ is alkyl, alkenyl, alkynyl, cycloalkyl, alkaryl, heteroarylalkyl or heterocyclylalkyl; and
R₅ can be hydrogen, alkyl, alkenyl, alkynyl, aryl, cycloalkyl, alkaryl, heteroaryl, heteroarylalkyl, heterocyclyl or heterocyclylalkyl.

9. A method for preparing a compound of Formula XXI, or its pharmaceutically acceptable salts, pharmaceutically acceptable solvates, enantiomers, diastereomers or N-oxides, comprising the steps of:
a. reacting a compound of Formula XVII 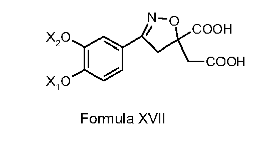 with a compound of Formula XVIIA
**Q** Formula XVIIA
to form a compound of Formula XVIII, 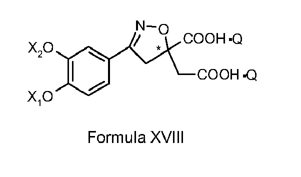
c. reacting the compound of Formula XVIII with a compound of Formula P'-OH to form a compound of Formula XIX, 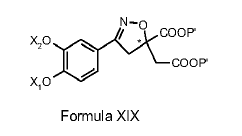
c. reducing the compound of Formula XIX to form a compound of Formula XX, and 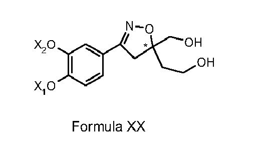
d. cyclizing the compound of Formula XX to form a compound of Formula XXI, 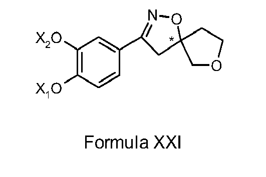
wherein
X₁ and X₂ is hydrogen, alkyl, cycloalkyl, alkaryl, cycloalkylalkyl, heteroaryl, heterocyclyl, heteroarylalkyl, heterocyclylalkyl, -SO₂R₅, -(CH₂)gNHCOOalkyl, -(CH₂)gC(=O)NRₓR_{y} or - (CH₂)_{g1}C(=O)OR₃ (wherein g is an integer from 0-3 and g₁ is an integer from 1-3);
X₁ and X₂ together optionally form a cyclic ring fused with ring A of Formula I, wherein ring A contains 3-5 carbon atoms and 2-3 heteroatoms selected from N, O or S;
Q is a chiral resolving agent; and
P' is alkyl.
